# EUROPÄISCHE PATENTSCHRIFT

(11) **EP 1 994 048 B1**
(45) Veröffentlichungstag und Bekanntmachung des Hinweises auf die Patenterteilung: **16.09.2009**
(21) Anmeldenummer: 07723281.7
(22) Anmeldetag: 15.03.2007
(51) Int. Cl.: C07K 14/33, A61K 38/16

(54) **PEGYLIERTES MUTIERTES CLOSTRIDIUM BOTULINUM TOXIN**
PEGYLATED MUTATED CLOSTRIDIUM BOTULINUM TOXIN
TOXINE MUTÉE ET PÉGYLÉE DE CLOSTRIDIUM BOTULINUM

(30) Priorität: 15.03.2006 EP 06005300
(43) Veröffentlichungstag der Anmeldung: 26.11.2008
(73) Patentinhaber: Biotecon Therapeutics GmbH, 14473 Potsdam (DE)
(72) Erfinder: FREVERT, Jürgen, 10625 Berlin (DE); SPECHT, Volker, 12207 Berlin (DE)
(74) Vertreter: Dehmel, Albrecht
(86) Internationale Anmeldenummer: PCT/EP2007/002296
(87) Internationale Veröffentlichungsnummer: WO 2007/104567

(56) Entgegenhaltungen:
- WO-A-03/000193
- WO-A-20/06026780
- WO-A2-00/74703
- SCHANTZ E J ET AL: "PROPERTIES AND USE OF BOTULINUM TOXIN AND OTHER MICROBIAL NEUROTOXINS IN MEDICINE" MICROBIOLOGICAL REVIEWS, AMERICAN SOCIETY FOR MICROBIOLOGY, WASHINGTON, DC, US, Bd. 56, Nr. 1, 1. März 1992 (1992-03-01), Seiten 80-99, XP000569909 ISSN: 0146-0749

## Beschreibung

Die vorliegende Erfindung betrifft modifizierte Botulinumtoxine (BoNT), die im Vergleich zu den entsprechenden nativen Botulinumtoxinen eine größere Stabilität und damit eine verlängerte therapeutischeWirkdauer aufweisen. Die vorliegende Erfindung betrifft weiterhin pharmazeutische Zusammensetzungen, die diese modifizierten Botulinumtoxine enthalten. Schließlich betrifft die vorliegende Erfindung Nukleinsäuren, die für diese modifizierten Botulinumtoxine kodieren.

### Hintergrund der Erfindung

*Clostridium botulinum* ist ein anaerob wachsendes, sporenbildendes Bakterium, das ein hochtoxisches Protein bildet. Dieses so genannte Botulinumtoxin ist die Ursache des Botulismus, einer Lebensmittelvergiftung, die ohne Einsatz von intensivmedizinischen Maßnahmen zum Tode des Botulismuspatienten führen kann. Man unterscheidet sieben Serotypen (Typ A-G, kurz bezeichnet als BoNT/A, BoNT/B, etc.), die eine ähnliche Aminosäure-Sequenz besitzen, aber eine unterschiedliche Antikörperantwort induzieren. Die Toxine (nachfolgend auch Neurotoxine und Botulinumtoxine genannt) bestehen aus zwei funktionellen Ketten, der Leichten (∼50 kD) und der Schweren Kette (∼ 100 kD), die bei einigen Clostridien-Stämmen durch proteolytische Spaltung des einzelkettigen Vorläufer-Proteins entstehen. Andere Stämme besitzen keine entsprechende Protease, die Spaltung in die Ketten erfolgt daher erst im Magen-Darm-Trakt des Patienten (z.B. durch Trypsin). In der zweikettigen Form sind die Untereinheiten (das heißt, die Schwere und die Leichte Kette) über eine Disulfid-Brücke miteinander verbunden (darüber hinaus besteht z.B. bei BoNT/A eine Disulfid-Brücke intramolekular, also zwischen zwei Cystein-Resten der Schweren Kette).

Die reinen Neurotoxine liegen *in vivo* unter sauren Bedingungen nicht frei vor, sondern bilden mit anderen clostridialen Proteinen Komplexe, die so genannten *(Clostridium botulinum)* Toxin-Komplexe. An diesen Komplexen sind verschiedene Proteine u. a. mit hämagglutinierenden Eigenschaften beteiligt. Die Zusammensetzung der Komplexe unterscheidet sich von Serotyp zu Serotyp. Die Integration in einen Komplex schützt das Neurotoxin bei der Magen-Darm-Passage. Möglicherweise spielen diese anderen clostridialen Proteine (die komplexierenden bzw.

Komplexproteine) auch bei der Resorption des Neurotoxins eine Rolle. Die Einbindung in den Komplex bewirkt also, dass das Neurotoxin oral bioverfügbar und damit ein Lebensmittelgift ist. Der Wirkort des Neurotoxins liegt an der motorischen Endplatte, wo der Muskel vom Nerv aktiviert wird. Das Motoneuron schüttet zur Aktivierung des Muskels Acetylcholin aus. Diese Ausschüttung wird durch Botulinumtoxin verhindert. Die inhibitorische Wirkung vollzieht sich in 3 sequentiellen Schritten: Bindung, Translokation, Proteolyse. Die Schwere Kette von Botulinumtoxin bindet hochspezifisch an das Motoneuron und wird anschließend von der Nervenzelle in Endosomen aufgenommen. Vor der Bindungsdomäne, die am C-Terminus der Schweren Kette lokalisiert ist, befindet sich im N-terminalen Abschnitt der Schweren Kette die Translokationsdomäne, die über einen bisher nicht genau bekannten Mechanismus die Leichte Kette in das Cytosol der Nervenzelle befördert bzw. die Translokation ermöglicht. Im Cytosol wird die Leichte Kette als Protease aktiv, sie spaltet hochspezifisch so genannte SNARE-Proteine. Die proteolytische Spezifität der einzelnen Botulinumtoxin-Typen ist in Tabelle 1 zusammengefasst. Diese SNARE-Proteine sind verantwortlich für die Fusion der Acetylcholinbeladenen sekretorischen Vesikel mit der Zellmembran des Motoneurons. Die proteolytische Spaltung eines dieser SNARE-Proteine verhindert die Bildung eines Fusionskomplexes und damit die weitere Ausschüttung von Acetylcholin. Der betroffene Muskel wird nicht mehr aktiviert. Vorher hyperaktive Muskeln werden paralysiert.

**Tabelle 1**

| **Botulinumtoxin-Typ** | **SNARE-Protein Substrat der Proteaseaktivität** | **Schnittstelle bei SNARE-Sequenz aus Ratte** |
|---|---|---|
| Typ A | SNAP 25 | EANQ¹⁹⁷ RATK |
| Typ B | VAMP 2 | GASQ⁷⁶ FETS |
| Typ C | Syntaxin SNAP 25 | DTKK²⁵⁴ AVKY ANQR¹⁹⁸ ATK |
| Typ D | VAMP 2 | RDQK⁶¹ LSED |
| Typ E | SNAP 25 | QIDR¹⁸⁰ IMEK |
| Typ F | VAMP 2 | ERDQ⁶⁰ KLSE |
| Typ G | VAMP 2 | ETSA⁸³ AKLK |

Dieser Wirkmechanismus wird bei der Therapie einer Vielzahl von Dystonien bzw. Spasmen ausgenutzt, die durch eine unkontrollierte Ausschüttung von Acetylcholin gekennzeichnet sind (z.B. Blepharospasmus, Torticollis, Spastizität). Zur Therapie der Dystonie werden äußerst geringe Mengen des Neurotoxins (im pg- bis ng-Bereich) in den hyperaktiven Muskel injiziert. Das Neurotoxin diffundiert zur motorischen Endplatte und gelangt in das Cytosol des Neurons, um dort die Acetylcholin-Ausschüttung zu verhindern. Nach 1-2 Tagen ist der Muskel paralysiert.

Verschiedene Gesichtsfalten entstehen durch die Verkrampfung von unter der Haut liegenden Muskeln, also ebenfalls durch eine ungeregelte Ausschüttung von Acetylcholin. Hier finden Botulinumtoxine ihre kosmetische Anwendung: durch Injektion von äußerst geringen Mengen von Botulinumtoxin lassen sich die Falten für ca. 3 Monate beseitigen.

Derzeit sind vier Präparate, die Botulinumtoxine enthalten, arzneimittelrechtlich zugelassen: Botox^{®} (Allergan), Xeomin^{®} (Merz), Dysport^{®} (Ipsen) und NeuroBloc^{®} (Solstice Neurosciences). Botox^{®}, Xeomin^{®} und Dysport^{®} sind Lyophilisate von Botulinumtoxin Typ A (als Komplex, Neurotoxin bzw. Komplex), Botox^{®} und Xeomin^{®} mit je 100 Einheiten pro Injektionsfläschchen, Dysport^{®} mit 500 Einheiten. NeuroBloc^{®} enthält Botulinumtoxin Typ B (als Komplex) mit 5.000 bzw. 10.000 Einheiten in flüssiger Formulierung.

Die Präparate liegen bis auf NeuroBloc als Lyophilisate vor, die mit Kochsalzlösung rekonstituiert und in je nach Präparat und Indikation abgestimmten Dosen in die betreffenden Muskeln injiziert werden. Innerhalb von 48 h wird der behandelte Muskel paralysiert. Die Wirkung hält ca. 3 Monate an, danach muss eine weitere Injektion erfolgen, wenn der Muskel weiterhin gelähmt bleiben, die Dystonie also behandelt werden soll. Bisher ist nicht eindeutig geklärt, welche Prozesse die Abnahme der Wirkung steuern. Solange die Leichte Kette als Protease aktiv ist, wird das entsprechende SNARE-Protein gespalten (z.B. SNAP 25 von der Leichten Kette vom Neurotoxin Typ A). Folglich wird unter diesen Bedingungen die Fusion der sekretorischen Vesikel mit der Plasmamembran und damit die Ausschüttung von Acetylcholin verhindert, der Muskel bleibt paralysiert. Gelänge es, die Protease-Aktivität der Leichten Kette in der Zelle für eine verlängerte Zeit aufrecht zu erhalten, so wäre auch die Wirkdauer eines entsprechenden Arzneimittels verlängert.

Im Unterschied zu vielen niedermolekularen Wirkstoffen sind Proteinwirkstoffe durch eine erheblich geringere Stabilität gekennzeichnet. Die Halbwertszeit (HWZ) in der Blutzirkulation beträgt bei einigen Proteinwirkstoffen z.B. nur wenige Minuten, so dass die (therapeutische) Wirkdauer stark eingeschränkt ist und in kurzen Abständen erneut injiziert werden muss. Die HWZ lässt sich verlängern, wenn es gelingt, das Protein vor Abbau- und Eliminierungsprozessen zu schützen. Ein theoretisch möglicher Weg besteht insbesondere bei eukaryontischen Proteinen in einer höheren Glykosylierung (mehr Kohlenhydrat-Reste) bzw. darin, die Kohlenhydrat-Strukturen den Strukturen humaner Glykoproteine anzupassen. Ein weitere Weg, der bei einer Reihe von zugelassenen Wirkstoffen beschritten wurde, besteht in der Verknüpfung des Proteins mit Polyethylenglykol (PEG). PEG kann kovalent an die Reste verschiedener Aminosäuren gebunden werden, z.B. an Lysin- (Amino-Funktion) oder Cystein-Reste (SH-Funktion). PEG erhöht das Molekulargewicht des Proteins, ohne dass immunogene Strukturen entstehen, die die Bildung von Antikörpern gegen den Wirkstoff induzieren. Im Gegenteil: die PEGylierung vermindert die Immunogenität des Wirkstoffes. Durch die Erhöhung des Molekulargewichts wird das Protein langsamer eliminiert und eine deutliche Erhöhung der HWZ im Serum erreicht. Um einen bestimmten erforderlichen Serumspiegel aufrecht zu erhalten, muss das Arzneimittel weniger häufig injiziert werden.

PEGylierte Protein-Wirkstoffe werden bereits in einigen zugelassenen Arzneimitteln verarbeitet (s. Tabelle 2). Der Einsatz von den z.T. kleinen Proteinen (z.B. Interferon α 2a: Mr = 19.3 kD) in der ursprünglichen Form, also nicht modifiziert, hatte gezeigt, dass die Proteine sehr schnell aus dem Serum eliminiert werden. Die PEGylierung führte zu einem deutlich erhöhten Molekulargewicht und damit zu einer wesentlich längeren Halbwertszeit im Serum. So beträgt z.B. bei Interferon α 2a die Serum-Halbwertszeit 9 h, die PEGylierung mit einer 40 kD PEG-Kette erhöht das Molekulargewicht drastisch und verlängert die Halbwertszeit von 9 auf 72 h.

**Tabelle 2**

| **Handelsname** | **Ausgangswirkstoff** | **Kopplung von PEG** |
|---|---|---|
| Pegasys | Interferon α 2a | verzweigtes PEG-N-Hydroxysuccinimid; Kopplung an 4 Lysin-Reste |
| Neulasta | G-CSF | PEG-Aldehyd; Methionin Kopplung an N-terminales Methionin |
| Peglutron | Interferon α 2b | Succinimidylcarbonat-PEG; Kopplung an Histidin- und Lysin-Reste |
| Somavest | Wachstumshormon-antagonist | 4 - 6 PEG; Kopplung an Lysin-Reste und N-Terminus |
| Oncaspar | Asparaginase | N-Hydroxysuccinimid aktiviertes PEG |

Die Verknüpfung mit einer oder mehreren PEG-Ketten unterliegt allerdings Beschränkungen:
1. Die PEG-Kette vermindert die biologische Aktivität des modifizierten Proteins (im Vergleich zum unmodifizierten nativen Protein) vorzugsweise gar nicht oder nur geringfügig (unter geringfügig verminderter biologischer Aktivität des modifizierten Proteins wird erfindungsgemäß eine biologische Aktivität des modifizierten Proteins verstanden, die wenigstens 20 %, vorzugsweise 30-40 % oder 50-70 % oder sogar 75-95 %, der biologischen der Aktivität des unmodifizierten nativen Proteins entspricht). Eine verminderte Aktivität ist in vielen Fällen aber durchaus tolerabel: die antivirale Aktivität von PEGyliertem Interferon beträgt z.B. 25-35 % des un-PEGylierten Interferon α 2b. PEGyliertes Interferon α 2a hat sogar nur 1-7 % der Aktivität der un-PEGylierten Form.
2. Da eine Vielzahl von therapeutisch eingesetzten Proteinen die Wirkung über die Bindung an einen spezifischen Rezeptor entfaltet, beeinflusst die PEGylierung die Wechselwirkung mit dem Rezeptor vorzugsweise nicht oder nur geringfügig (die Wechselwirkung kann z.B. durch sterische Hinderung direkt an der Bindungsdomäne oder durch Veränderungen der Raumstruktur des Proteins, die sich auf die Bindungsdomäne und somit auf die Bindung auswirken, beeinträchtigt werden).
3. Wird die pharmakologische Wirkung des therapeutischen Proteins (auch) über eine enzymatische Aktivität vermittelt (wie z.B. bei der Asparaginase), drosselt die PEGylierung die enzymatische Aktivität vorzugsweise nicht oder nur geringfügig.

Die PEGylierung von Botulinumtoxin erfüllt vorzugsweise diese drei Kriterien. Dabei beeinflusst die Modifizierung des Botulinumtoxins mit PEG vorzugsweise weder (a) die Bindungsdomäne der Schweren Kette noch (b) die enzymatische Aktivität der Leichten Kette, d.h. die PEG-Kette inhibiert die Wechselwirkung der katalytischen Domäne auf der Leichten Kette mit dem Substrat (SNARE-Protein) vorzugsweise nicht. Im Unterschied zu anderen Proteasen, die kurze Peptide spalten, erfordern Botulinumtoxine längere Peptide als Substrate. So hat ein Peptid, das dem Botulinumtoxin Typ B als Substrat dient, vorzugsweise eine Sequenz von etwa 40 Aminosäure-Resten aus dem SNARE-Protein VAMP 2. peptide mit kürzeren SNARE-Sequenzen werden zwar auch gespalten, dies aber mit erheblich geringerer Effizienz. Die Spaltungsdomäne auf der Leichten Kette des Botulinumtoxins, die eine der Erkennungssequenz von etwa 40 Aminosäure-Resten vergleichbare Länge hat, wird vorzugsweise von der PEG-Kette nicht beeinflusst. Zu berücksichtigen ist ferner, dass außer der Spaltungsdomäne, die für den direkten Kontakt des Substrats (SNARE-Protein bzw. Peptid mit der SNARE-Sequenz von etwa 40 Aminosäure-Resten) mit der Leichten Kette verantwortlich ist, noch weitere Kontaktstellen mit entfernt von der katalytischen Domäne auf der Leichten Kette liegenden Sequenzen für die optimale Aktivität von Botulinumtoxin erforderlich sind. So wurde nachgewiesen, dass es auf der Leichten Kette von Botulinumtoxin Typ A fünf weitere Kontaktstellen für sein Substrat SNAP 25 gibt: 4 "α exosites" (AS 102-113, 310-321, 335-348, 351-358) und eine "β exosite" (AS 242-259). Der Kontakt wird vorzugsweise durch eine Konjugation der Leichten Kette mit PEG nicht oder nur unwesentlich behindert. Ferner muss der C-terminale Teil der Schweren Kette, die Translokationsdomäne, funktions-tüchtig sein, d.h. dafür sorgen, dass die Leichte Kette aus den Endosomen in das Cytosol befördert wird. Dieser für die Wirkung unabdingbare Transportprozess kann auch durch sterische Hinderungen einer PEGylierten Leichten Kette gehemmt werden, zumal die Translokationsdomäne möglicherweise eine Pore in der Endosomen-Membran bildet, durch die eine "sperrige" PEGylierte Leichte Kette nicht hindurchgeschleust werden könnte.

In einer US-Patentanmeldung (2002/0197278) wird eine Kopplung von PEG an Botulinumtoxin beschrieben. Die Kopplung dient der Verminderung der Antigenität bzw. Immunogenität sowie der Erhöhung des Molekulargewichts zur Reduktion der Diffusion. Zur Auswahl der geeigneten Stellen (antigenen Determinanten) bzw. Aminosäure-Reste für die PEGylierung wird auf die Arbeit von Bavari et al. (Vaccine 16: 1850-1856, 1998) verwiesen. In dieser Arbeit werden Sequenzen der Schweren Kette von Botulinumtoxin dargestellt, die neutralisierende Antikörper induzieren. In der genannten Patentanmeldung wird lediglich angegeben, dass (1) die PEGylierung an bzw. nahe einer Stelle oder an bzw. nahe den Stellen erfolgen soll, die als (ein) wichtige(s) Epitop(e) wirkt/en, aber entfernt von der katalytischen Domäne (also entfernt von der Leichten Kette) ist/sind, und dass (2) PEG an die freien endständigen Carboxy- oder Aminogruppen oder an die Aminogruppen von Lysin-Seitenketten konjugiert werden kann. (3) Als weitere Möglichkeit, PEG in das Toxin einzuführen, wird vorgeschlagen, SH-Gruppen natürlicherweise vorkommender oder extra eingeführter Cystein-Reste zu verwenden, wobei allerdings vor dieser Variante (3) gewarnt wird, da Disulfid-Brücken zwischen der Schweren und Leichten Kette des Botulinumtoxins bei der räumlichen Gestaltung des Moleküls eine Rolle spielen. Es wird kein Beispiel aufgeführt, aus dem die Struktur des PEGylierten Neurotoxins hervorgeht oder an welchem/n Aminosäure-Rest(en) ein PEG-Molekül welcher Länge angebracht wurde.

In einer weiteren Patentanmeldung (WO 02/40506), die sich auf die Änderung der Persistenz bezieht, wird die Einführung, die Abänderung oder die Entfernung von Stellen für die *in vivo-*Glykosylierung, *in vivo*-Phosphorylierung und vor allem die in vivo-Myristylierung im Botulinumtoxin vorgeschlagen, um die Stabilität des Botulinumtoxins wahlweise zu erhöhen oder zu verringern. Es werden eine ganze Reihe solcher potentiellen Modifizierungsstellen angegeben, die mit einem deutlichen Abstand zu den N- und C-terminalen Enden auf der leichten Kette des Neurotoxins liegen. Dazu sollen zusätzlich Sequenzen in die Polypeptid-Kette eingebaut werden, an denen zelluläre Enzyme Kohlenhydrat-Ketten bzw. Phosphat- bzw. Myristylat-Reste an die Leichte Kette koppeln. Angaben zu einem entsprechend modifizierten Neurotoxin bzw. zu seiner Herstellung fehlen jedoch.

In einer weiteren US-Patentanmeldung (2003/0027752) wird in das Neurotoxin bzw. in die Leichte Kette ein Peptid-Rest mit einem so genannten Leucin-Motiv eingefügt (z.B. XEXXXLL), um die Persistenz der Leichten Kette in der Nervenzelle zu erhöhen. Durch die Ausstattung der Leichten Kette mit diesem Motiv soll erreicht werden, dass sie in der Membran in der Nähe ihres Substrats lokalisiert wird. Des weiteren wird ein so genanntes "Tyrosine-based Motive" (YXXHy, Y = Tyrosin, Hy = hydrophobe Aminosäure) angegeben, das nach Einfügung in die Leichte Kette deren Persistenz erhöhen soll. Schließlich schlägt diese Anmeldung ein modifiziertes Botulinumtoxin Typ A vor, in dem die Leichte Kette mutiert ist (jeweils von Alanin nach Leucin an den Positionen 427 und 428).

Die Aufgabe, die sich die Erfinder angesichts des vorstehend beschriebenen Standes der Technik gestellt haben, bestand also darin, eine weitere bzw. konkret beschriebene Form der Stabilisierung von Botulinumtoxin jeglichen Serotyps, vorzugsweise jedoch des Typs A, B und C1, bereitzustellen. Damit einhergehend bestand die Aufgabe der Erfinder also darin, stabile Varianten/Analoga der natürlichen Botulinumtoxine zur Verfügung zu stellen, die im Vergleich zu den entsprechenden unmodifizierten Botulinumtoxinen eine erhöhte Stabilität *in vivo* aufweisen. Das heißt erstens, dass die biologische Aktivität (erfindungsgemäß wird biologische Aktivität definiert als Gesamtaktivität, umfassend die enzymatische/katalytische Aktivität der Leichten Kette sowie die dafür erforderliche Bindung des Neurotoxins an die Zielzelle und die Translokation der Leichten Kette in die Zielzelle) der/s Botulinumtoxin-Variante/Analogen allenfalls geringfügig (gemäß Definition oben) und vorzugsweise gar nicht vermindert sein soll, und zweitens, dass die Leichte Kette trotz ihrer Modifikation an ihren Wirkort, in das Cytosol der Motoneuronen, transloziert wird.

Im Unterschied zu der oben schon genannten US 20020197278 stellt die Aufgabe, die der vorliegenden Patentanmeldung zu Grunde liegt, also nicht darauf ab, antigene Determinanten zu blockieren, die Antigenität der Toxine zu vermindern oder deren Diffusion weg vom Ort der Injektion einzuschränken.

Überraschenderweise haben die Erfinder der vorliegenden Anmeldung gefunden, dass die Leichte Kette von Botulinumtoxinen an ihrem N-Terminus mittels Einführung von mindestens einem Cystein-Rest spezifisch PEGyliert werden kann, ohne dass gleichzeitig die biologische Aktivität (gemäß vorstehend gegebener Definition) der Botulinumtoxine beeinträchtigt oder gar gehemmt wird. Ein derart PEGyliertes Botulinumtoxin zeichnet sich durch eine überraschend höhere *in vivo*-Stabilität (deutliche Erhöhung der HWZ und damit eine verlängerte (pharmakologische) Wirkdauer aus.

Die (therapeutische) Wirkdauer der natürlichen Botulinumtoxine im Patient ist abhängig vom Serotyp. Botulinumtoxin Typ A zeichnet sich durch die längste Wirkdauer von ca. 3 Monaten aus. Botulinumtoxin Typ C wirkt etwa gleich lang wie Typ A, während Botulinumtoxin Typ B etwas kürzer wirkt. Die Wirkung von Botulinumtoxin Typ E und F beträgt jeweils nur ca. 2 Wochen. Die kurze Wirkdauer dieser beiden Typen erlaubt ihre klinische Anwendung zur Behandlung von Dystonien nicht. Die vorliegende Erfindung ermöglicht (1) den klinischen Einsatz aller Botulinumtoxine, auch der mit bisher nur kurzzeitiger Wirkung, und (2) eine vorteilhaftere Therapie mit den bereits therapeutisch eingesetzten Toxinen der Typen A und B, da diese nicht mehr vierteljährlich, sondern z.B. nur noch halbjährlich appliziert werden müssen.

### Beschreibung der Abbildungen und Sequenzen

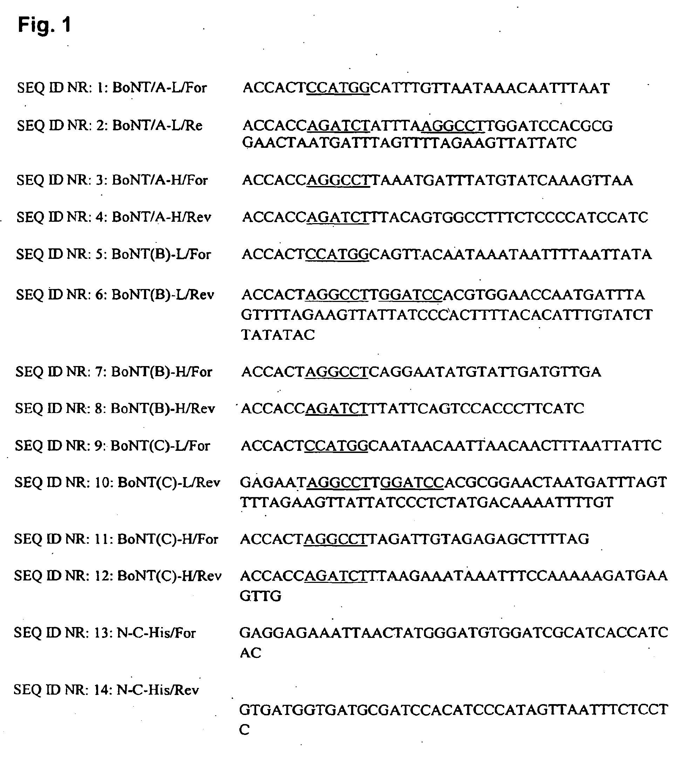

Fig. 1: Zusammenstellung der Oligonukleotide (SEQ ID NR:1 bis 14), die bei den Klonierungen der rekombinanten Toxine und Toxin-Fragmente eingesetzt wurden. Erkennungssequenzen für Restriktionsendonukleasen sind unterstrichen. Die langen Sequenzen mit den SEQ ID NR:16 und 15 stellen Beispiele für ein rekombinantes (mutiertes) Botulinum-Neurotoxin Typ A mit angefügtem Cystein-Rest N-terminal von einem Histidin-Tag (bestehend aus 10 Histidin-Resten) bzw. eine DNA, die für dieses kodiert, dar. Der Prolin-Rest am N-Terminus des monocistronisch exprimierten und translatierten nativen Toxins (Position 1) wurde durch einen Alanin-Rest ersetzt, um eine Schnittstelle in der Multi-Cloning Site (MCS) des Vektors zu schaffen. Der Vektor umfasst die für den His-Tag kodierende Sequenz nämlich exakt in 5'-Nachbarschaft zu dieser MCS. Außerdem wurde statt des nativen Loops zwischen der Leichten und Schweren Kette bereits eine Sequenz eingeführt, die von E. coli-Zellen erkannt wird, woraufhin das native Präpeptid (N-Leichte Kette-Loop-Schwere Kette-C) bereits bei der rekombinanten Herstellung des Neurotoxins und ohne Zusatz exogener Proteasen in das aktive zweikettige Neurotoxin gespalten und als solches erhalten wird.

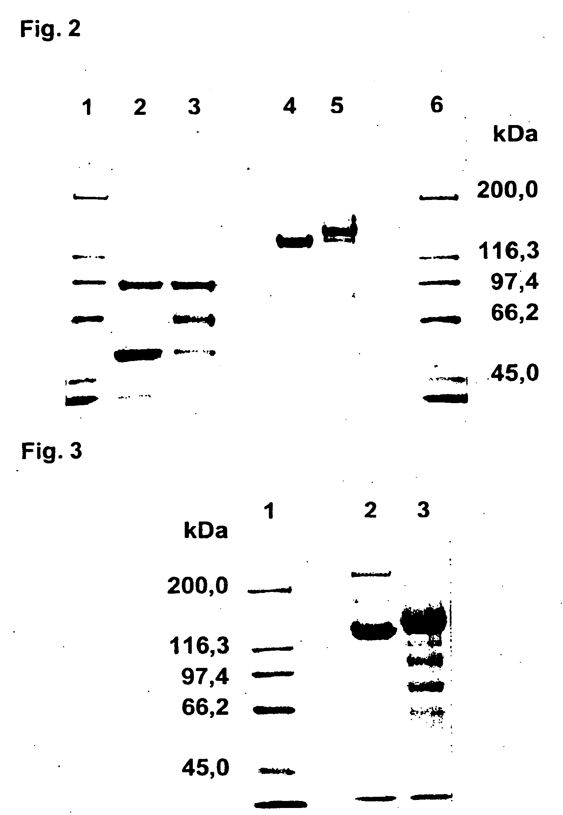

Fig. 2: Analyse des PEGylierungs- und Kontrollansatzes von C-H₁₀-BoNT/A (Beispiel 5) im SDS-Polyacrylamidgel unter nicht-reduzierenden Bedingungen. Spur 1: Molekulargewichtsmarker; Spur 2: Kontrollansatz; Spur 3: PEGylierungsansatz.

### Beschreibung der Erfindung

Um die gestellte Aufgabe (siehe oben) zu lösen, haben die Erfinder modifizierte Botulinumtoxine entwickelt. Ein Aspekt und Gegenstand der Erfindung ist somit ein modifiziertes Botulinumtoxin, das eine natürliche Schwere Kette und eine modifizierte Leichte Kette aufweist, wobei die Modifikation der Leichten Kette darin besteht, (1) dass sie an ihrem N-Terminus eine Verlängerung der Kette aufweist, die in der Richtung vom N- zum C-terminalen Ende die folgende Struktur hat: -(C)ₙ-(tag)ₘ-(X)ₖ-,
wobei
C für einen Cystein-Rest,
tag für einen beliebigen Tag, z.B. für einen Strep-Tag oder einen His-Tag und
X für den Rest einer beliebigen natürlich vorkommenden Aminosäure stehen;
n eine ganze Zahl von 1 bis 50 ist;
m = 0 oder m = 1 und
k = 0 oder k eine ganze Zahl von 1 bis 50 ist,
und (2) dass wenigstens einer der Cystein-Reste in der Verlängerung der Kette an wenigstens eine Kette PEG gekoppelt ist. Ein derart modifiziertes Botulinumtoxin wird nachfolgend auch PEGyliertes mutiertes Botulinum- oder Neurotoxin genannt.

Gemäß einer bevorzugten Ausführungsform gelten folgende Bedingungen:
n = 1, 2 oder 3, m = 0 oder 1, k = 0 oder 1 # 0,
n = 1, m = 1, k = 0; n = 2, m = 1, k = 0 ; n = 3, m = 1, k = 0;
n = 1, m = 0, k = 0; n = 2, m = 0, k = 0; n = 3, m = 0, k = 0;
n = 1, m = 1, k ≠ 0; n = 2, m = 1, k ≠ 0; n = 3, m = 1, k ≠ 0;
n = 1, m = 0, k ≠ 0; n = 2, m = 0, k ≠ 0; n = 3, m = 0, k ≠ 0,
wobei die Toxine pro Molekül an eine, an zwei oder an drei PEG-Moleküle gekoppelt sind, je nachdem, ob n = 1, 2 oder 3.

Somit fallen unter die oben genannten modifizierten Botulinumtoxine der vorliegenden Erfindung vorzugsweise solche modifizierten Botulinumtoxine (insbesondere der Typen A, B und C1), deren Leichte Kette so modifiziert ist, dass diese an ihrem N-Terminus eine Verlängerung der Kette aufweist, wobei die verlängerte Kette eine der folgenden Sequenzen hat:
-(C)₁-(tag)₁-(X)₀-, -(C)₂₋(tag)₁-(X)₀-, -(C)₃-(tag)₁-(X)₀-, -(C)₄-(tag)₁-(X)₀-, -(C)₅-(tag)₁-(X)₀-, -(C)₁-(tag)₁-(X)₁-, -(C)₂-(tag)₁-(X)₁-, -(C)₃-(tag)₁-(X)₁-, -(C)₄-(tag)₁-(X)₁-, -(C)₅-(tag)₁-(X)₁-, -(C)₁-(tag)₁-(X)₂-, -(C)₂-(tag)₁-(X)₂-, -(C)₃-(tag)₁-(X)₂-, -(C)₄-(tag)₁-(X)₂-, -(C)₅-(tag)₁-(X)₂-, -(C)₁-(tag)₁-(X)₃-, -(C)₂-(tag)₁-(X)₃-, -(C)₃-(tag)₁-(X)₃-, -(C)₄-(tag)₁-(X)₃-, -(C)₅-(tag)₁-(X)₃-, -(C)₁-(tag)₁-(X)₄-, -(C)₂-(tag)₁-(X)₄-, -(C)₃-(tag)₁-(X)₄-, -(C)₄-(tag)₁-(X)₄-, -(C)₅-(tag)₁-(X)₄-, etc. wobei für jede der vorstehend aufgelisteten 25 bevorzugten Ausführungsformen (tag)₁ auch (tag)₀ sein kann, und/oder jeweils alle in der Verlängerung der Leichten Kette vorkommenden Cystein-Reste auch PEGyliert sind.

Denkbar ist dass k auch jede beliebige ganze Zahl von 11 bis 50, oder auch über 50, insbesondere über 100 oder über 250 ist. Je größer k aber ist, umso länger wird die Leichte Kette, ohne dass durch eine bestimmte Obergrenze der Länge der Leichten Kette deren enzymatische/katalytische Aktivität bzw. die biologische (Gesamt-) Aktivität (gemäß vorstehender Definition) des Neurotoxins gefährdet wäre. Erfindungsgemäß bietet sich eine obere Grenze für k von 10-20 an, so dass bevorzugte Werte für k im Bereich von 1-10 liegen, sofern k nicht, besonders bevorzugt, 0 ist.

Entsprechende Überlegungen treffen auch auf n zu, wobei dessen Obergrenze erfindungsgemäß einzig aus praktischen und ökonomischen Gründen auf 50 festgelegt wurde. Vorzugsweise ist n jedoch im Bereich von 1-10, besser 1-5, um nicht zu viele PEG-Moleküle einzuführen bzw. einführen zu müssen (da vorzugsweise alle eingeführten Cystein-Reste auch PEGyliert werden) und das resultierende PEGylierte mutierte Botulinum-/Neurotoxin nicht seiner biologischen Aktivität gemäß vorstehender Definition zu berauben. Dies kann leicht geschehen, wenn zu viele Cystein- und PEG-Reste bzw. wenn zu viele Cystein- und PEG-Reste an falschen Positionen eingeführt werden, da die Leichte Kette, ggf. trotz Bindung des Toxins an die Zielzelle, nicht in die Zielzelle transloziert wird.

Die Struktur von Botulinumtoxin Typ A wurde von Lacy & Stevens 1998 publiziert (Nat. Struct. Biol. 5, 898-902), die Struktur von Botulinumtoxin Typ B von Swaninathan & Eswaramoorthy (Nat. Struct. Biol 7, 693-699 (2000)). Damit ist auch die Struktur der Leichten Ketten bekannt, und es lässt sich ermitteln, welche Bereiche der Schweren bzw. Leichten Kette an der Proteinoberfläche liegen und deshalb für eine Verknüpfung mit PEG geeignet sein können. Die häufig gewählte Vorgehensweise, die Bindung eines geeignet aktivierten PEG (z.B. PEG-Succinimidylpropionat) an die ε-Aminogruppe von Lysin-Resten erschien den Erfindern nicht erfolgversprechend. Ein aktiviertes PEG kann an zahlreichen Lysin-Resten angreifen - auch im Bindungsbereich der Schweren Kette, was experimentell zu einer drastischen Inaktivierung führte.

Stattdessen haben die Erfinder Aminosäure-Reste der Leichten Kette identifiziert, die sich dazu eignen, zunächst durch mindestens einen Cystein-Rest ausgetauscht und anschließend an dem mindestens einen eingeführten Cystein-Rest PEGyliert zu werden. Auch diese, nachfolgend noch näher zu charakterisierenden, modifizierten Botulinumtoxine sind eine bevorzugte Ausgestaltung der vorliegenden Erfindung, weisen als Konjugate mit PEG eine ausreichende biologische (inklusive enzymatische/katalytische) Aktivität (die definitionsgemäß wenigstens 20 %, vorzugsweise 30-40 %, 50-70 % oder sogar 75-95 % der biologischen Aktivität des unmodifizierten Proteins entspricht) bei gleichzeitig erhöhter Stabilität (im Vergleich zu den entsprechenden nativen Neurotoxinen) auf und werden nachfolgend ebenfalls als PEGylierte mutierte Botulinum- oder Neurotoxine der vorliegenden Erfindung bezeichnet.

Diese letztgenannten modifizierten Botulinumtoxine der vorliegenden Erfindung sind ebenfalls Konjugate von mutierten Botulinumtoxinen mit PEG. Auch diese modifizierten Botulinumtoxine sind über gesondert eingeführte Cystein-Reste an PEG gekoppelt. Dazu wird mindestens einer, ggf. aber auch 2, 3, 4, 5, 10 oder sogar alle 20, der ersten 20 Aminosäure-Reste vom N-terminalen Ende der Leichten Kette des entsprechenden Botulinumtoxins jeweils durch ein Cystein-Rest ersetzt. Diese modifizierten Botulinumtoxine weisen ebenfalls eine natürliche Schwere Kette und eine modifizierte Leichte Kette auf, wobei die Modifikation der Leichten Kette eben darin besteht, dass mindestens ein bis maximal 20 der natürlicherweise an ihrem N-Terminus sitzenden Aminosäure-Reste zu einem Cystein-Rest mutiert ist bzw. sind. Ggf. weisen sie zusätzlich eine N-terminale Verlängerung auf, so dass die Sequenz der Leichten Kette in der Richtung vom N- zum C-terminalen Ende die folgende Struktur hat:
- (tag)ₘ (X)ₖ-BoNT(X1-20C),
wobei
C für einen Cystein-Rest,
tag für einen beliebigen Tag, z.B. für einen Strep-Tag oder einen His-Tag und
X für den Rest einer beliebigen natürlich vorkommenden Aminosäure stehen;
m = 0 oder m = 1 und
k = 0 oder k eine ganze Zahl von 1 bis 50 ist.
Wenigstens einer der maximal 20 Cystein-Reste am N-Terminus ist an wenigstens eine Kette PEG gekoppelt.

Für das BoNT/A ergeben sich somit Mutanten, die wenigstens durch eine - und höchstens zwanzig - der nachfolgenden Aminosäure-Rest-Austausche gekennzeichnet sind, so dass an den eingeführten Cystein-Resten eine PEGylierung erfolgen kann:
P1C, F2C, V3C, N4C, K5C, Q6C, F7C, N8C, Y9C, K10C, D11C, P12C, V13C, N14C, G15C, V16C, D17C, I18C, A19C, Y20C

Gemäß einer bevorzugten Ausführungsform gelten folgende Bedingungen:
m = 1, k = 0, nur einer der 20 N-terminalen Aminosäure-Reste ist durch einen Cystein-Rest ersetzt, insbesondere nur der Rest an Position 1, 2, 3, 4, 5, 6, 7, 8, 9, oder 10;
m = 0, k = 0, nur einer der 20 N-terminalen Aminosäure-Reste ist durch einen Cystein-Rest ersetzt, insbesondere nur der Rest an Position 1, 2, 3, 4, 5, 6, 7, 8, 9, oder 10;
m = 1, k ≠ 0, nur einer der 20 N-terminalen Aminosäure-Reste ist durch einen Cystein-Rest ersetzt, insbesondere nur der Rest an Position 1, 2, 3, 4, 5, 6, 7, 8, 9, oder 10;
m = 0, k ≠ 0, nur einer der 20 N-terminalen Aminosäure-Reste ist durch einen Cystein-Rest ersetzt, insbesondere nur der Rest an Position 1, 2, 3, 4, 5, 6, 7, 8, 9, oder 10;
m = 1, k = 0, nur zwei der 20 N-terminalen Aminosäure-Reste sind durch einen Cystein-Rest ersetzt, insbesondere nur die Reste an den Positionen 1 und 3, 1 und 4, 2 und 4, 1 und 5, 2 und 5, 3 und 5, 1 und 6, 2 und 6, 3 und 6 oder 4 und 6;
m = 0, k = 0, nur zwei der 20 N-terminalen Aminosäure-Reste sind durch einen Cystein-Rest ersetzt, insbesondere nur die Reste an den Positionen 1 und 3, 1 und 4, 2 und 4, 1 und 5, 2 und 5, 3 und 5, 1 und 6, 2 und 6, 3 und 6 oder 4 und 6;
m = 1, k ≠ 0, nur zwei der 20 N-terminalen Aminosäure-Reste sind durch einen Cystein-Rest ersetzt, insbesondere nur die Reste an den Positionen 1 und 3, 1 und 4, 2 und 4, 1 und 5, 2 und 5, 3 und 5, 1 und 6, 2 und 6, 3 und 6 oder 4 und 6;
m = 0, k ≠ 0, nur zwei der 20 N-terminalen Aminosäure-Reste sind durch einen Cystein-Rest ersetzt, insbesondere nur die Reste an den Positionen 1 und 3, 1 und 4, 2 und 4, 1 und 5, 2 und 5, 3 und 5, 1 und 6, 2 und 6, 3 und 6 oder 4 und 6;
m = 1, k = 0, nur zwei der 20 N-terminalen Aminosäure-Reste sind durch einen Cystein-Rest ersetzt, insbesondere nur die Reste an den Positionen 1 und 2, 2 und 3, 3 und 4, 4 und 5 oder 5 und 6;
m = 0, k = 0, nur zwei der 20 N-terminalen Aminosäure-Reste sind durch einen Cystein-Rest ersetzt, insbesondere nur die Reste an den Positionen 1 und 2, 2 und 3, 3 und 4, 4 und 5 oder 5 und 6;
m = 1, k ≠ 0, nur zwei der 20 N-terminalen Aminosäure-Reste sind durch einen Cystein-Rest ersetzt, insbesondere nur die Reste an den Positionen 1 und 2, 2 und 3, 3 und 4, 4 und 5 oder 5 und 6;
m = 0, k ≠ 0, nur zwei der 20 N-terminalen Aminosäure-Reste sind durch einen Cystein-Rest ersetzt, insbesondere nur die Reste an den Positionen 1 und 2, 2 und 3, 3 und 4, 4 und 5 oder 5 und 6,
wobei die Toxine pro Molekül an ein oder an zwei PEG-Moleküle gekoppelt sind, je nachdem, ob nur ein oder zwei Aminosäure-Reste durch (einen) Cystein-Rest(e) N-terminal ersetzt wurden.

Somit fallen unter die oben genannten modifizierten Botulinumtoxine der vorliegenden Erfindung vorzugsweise solche Botulinumtoxine (insbesondere der Typen A, B und C1), deren Leichte Kette so modifiziert ist, dass diese an ihrem N-Terminus eine Verlängerung der Kette aufweist, wobei der N-Terminus der verlängerten Leichten Kette eine der folgenden Sequenzen aufweist:
-(tag)₁-(X)₀-BoNT(P1C), -(tag)₁-(X)₀-BoNT(F2C), -(tag)₁-(X)₀-BoNT(V3C), -(tag)₁-(X)₀-BoNT(N4C), (tag)₁-(X)₀-BoNT(K5C), -(tag)₁-(X)₁-BoNT(P1C), -(tag)₁-(X)₁-BoNT(F2C), -(tag)₁-(X)₁-BoNT(V3C), -(tag)₁-(X)₁-BoNT(N4C), -(tag)₁-(X)₁-BoNT(K5C), -(tag)₁-(X)₂-BoNT(P1C), -(tag)₁-(X)₂-BoNT(F2C), -(tag)₁-(X)₂-BoNT(V3C), -(tag)₁-(X)₂-BoNT(N4C), -(tag)₁-(X)₂-BoNT(K5C), -(tag)₁-(X)₃-BoNT(P1C), -(tag)₁-(X)₃-BoNT(F2C), -(tag)₁-(X)₃-BoNT(V3C), -(tag)₁-(X)₃-BoNT(N4C), -(tag)₁-(X)₃-BoNT(K5C), -(tag)₁-(X)₄-BoNT(P1C), -(tag)₁-(X)₄-BoNT(F2C), -(tag)₁-(X)₄-BoNT(V3C), -(tag)₁-(X)₄-BoNT(N4C), -(tag)₁-(X)₄-BoNT(K5C), etc.
wobei für jede der vorstehend aufgelisteten 25 bevorzugten Ausführungsformen (tag)₁ auch (tag)₀ sein kann, und/oder jeweils alle N-terminal eingeführten Cystein-Reste auch PEGyliert sind.

Denkbar ist dass k auch jede beliebige ganze Zahl von 11 bis 50, oder auch über 50, insbesondere über 100 oder über 250 ist Je größer k aber ist, umso länger wird die Leichte Kette, ohne dass durch eine bestimmte Obergrenze der Länge der Leichten Kette deren enzymatische/katalytische Aktivität bzw. die biologische (Gesamt-)Aktivität des Neurotoxins im Sinne der vorstehenden Definition gefährdet wäre. Erfindungsgemäß bietet sich eine obere Grenze für k von 10-20 an, so dass bevorzugte Werte für k im Bereich von 1-10 liegen, sofern k nicht, besonders bevorzugt, 0 ist.

Die folgenden Ausführungen gelten, sofern nicht explizit anders angegeben, für die modifizierten Botulinumtoxine der vorliegenden Erfindung, unabhängig davon, ob es sich um die Variante mit eingeführtem/n Cystein-Rest/en in der N-terminalen Verlängerung der Leichten Kette oder um die Variante mit eingeführtem/n Cystein-Rest/en am N-terminalen Ende der Leichten Kette handelt.

Vorzugsweise ist das modifizierte Botulinumtoxin ein von BoNT/A, BoNT/B oder BoNT/C1 abgeleitetes modifiziertes Botulinumtoxin, genauso kann es aber auch ein Botulinumtoxin der Typen D, E, F oder G sein. Ebenfalls bevorzugt ist, wenn einerseits alle künstlich eingeführten Cystein-Reste (vorzugsweise werden 1-10 Cystein-Reste eingeführt) mindestens eine PEG-Kette aufweisen, andererseits aber keiner der natürlicherweise in der Schweren und Leichten Kette des Botulinumtoxins vorkommenden Cystein-Reste PEGyliert ist.

Der Fachmann versteht natürlich ohne weiteres, dass die Bedeutung des Tags (m = 1) in der leichteren Aufreinigung des rekombinant (z.B. in *E*. *coli*) hergestellten modifizierten Botulinumtoxins der vorliegenden Erfindung liegt. Der Tag wird also nicht benötigt, um die Stabilität des Neurotoxins oder dessen biologische Aktivität zu erhöhen, sondern erlaubt die vereinfachte und nahezu quantitative Isolierung des modifizierten Botulinumtoxins aus der Bakterien-Kultur.

Bei der Frage nach einer geeigneten Wahl von n (im Fall der Variante mit eingeführtem/n Cystein-Rest/en in der N-terminalen Verlängerung der Leichten Kette) bzw. nach der Anzahl und Position der durch Cystein-Reste zu ersetzenden Aminosäure-Reste (im Fall der Variante mit eingeführtem/n Cystein-Rest/en am N-terminalen Ende der Leichten Kette) ist zu berücksichtigen, dass vorzugsweise nur so viele Cystein-Reste eingeführt werden wie PEGylierungen erfolgen sollen (entsprechendes gilt auch für den Fall, dass k ≠.0 und mindestens ein Aminosäure-Rest X ein Cystein-Rest ist). Diese eingeführten Cystein-Reste lassen sich vorzugsweise alle, und vollständig, PEGylieren, ohne dass, und dies ist die überraschende Erkenntnis der Erfinder, auch nur einer der natürlicherweise im Botulinumtoxin vorkommenden Cystein-Reste, weder auf der Schweren noch auf der Leichten Kette, PEGyliert wurde (neben je einer intra- und intermolekularen Disulfid-Brücke besitzt z.B. Botulinumtoxin Typ A noch drei weitere Cystein-Reste in der Schweren Kette (C₇₉₁, C₉₆₇, C₁₀₆₀) sowie zwei weitere Cystein-Reste in der Leichten Kette (C₁₃₄ und C₁₆₅)). Auf diese Weise lässt sich ein einheitliches (homogenes) Produkt in Form eines PEGylierten mutierten Botulinumtoxins erhalten. Darüber hinaus ist es aus einem weiteren Grund sinnvoll zu vermeiden, dass zu viele Cystein- und PEG-Reste bzw. dass zu viele Cystein- und PEG-Reste an falschen Positionen eingeführt werden. Dies deshalb, da (i) das Toxin dann möglicherweise zu sperrig wird, um an die Zielzelle zu binden und/oder (ii) die Leichte Kette, ggf. trotz Bindung des Toxins an die Zielzelle, nicht oder nicht in ausreichendem Maße in die Zielzelle transloziert wird. Mit anderen Worten, die Einführung nur *eines* Cystein-Restes, bei dieser oder jener Variante, und dessen PEGylierung erfüllt den erfindungsgemäßen Zweck regelmäßig und ist daher besonders bevorzugt.

Die PEGylierten mutierten Botulinum-/Neurotoxine gemäß der vorliegenden Erfindung sind folgerichtigerweise biologisch und enzymatisch (d.h. katalytisch) aktiv wie die entsprechenden natürlichen (nativen) Botulinum-/Neurotoxine oder weisen im Sinne der vorstehend gegebenen Definition höchstens geringfügig verminderte biologische und enzymatische/katalytische Aktivität auf, sind dabei aber stabiler, z.T. sogar erheblich stabiler, als ihre natürlichen Vorgänger-Toxine, von denen sie abgeleitet wurden. So ist weiterhin ein PEGyliertes mutiertes Botulinumtoxin bevorzugt, dessen PEGylierte mutierte (modifizierte) Leichte Kette im Cytosol der Motoneuronen eine höhere Stabilität aufweist als die unmodifizierte Leichte Kette des entsprechenden nativen Botulinumtoxins.

Die PEGylierung der Leichten Kette, wie vorstehend beschrieben, führt zu einer erhöhten Stabilität im Vergleich zur unmodifizierten Leichten Kette. Die PEG-Kette ist erfindungsgemäß also so an der Leichten Kette angebracht, dass 1. ihre enzymatische Aktivität unverändert oder allenfalls geringfügig vermindert ist (gemäß der oben gegebenen Definition), und 2. die modifizierte Leichte Kette, wie auch die unmodifizierte Kette, in das Cytosol der Motoneuronen transloziert wird.

Der His-Tag, wie auch andere Tags, z.B. der Strep-Tag, ermöglichen die einfache Isolierung des mutierten Neurotoxins aus dem Lysat transformierter Bakterien (z.B. *E*. *coli*). Er wird am einfachsten auf DNA-Ebene in 5'-Richtung des kodierenden Bereichs des Neurotoxin-Gens angebracht. Im Falle des His-Tags erfolgt die Isolierung mittels Affinitätschromatographie über Ni-NTA-Sepharose. So isoliertes mutiertes Neurotoxin wird im nächsten Schritt mit aktiviertem PEG gekoppelt. Die Fa. Nektar Therapeutics stellt eine Reihe von aktivierten PEG-Derivaten zur Verfügung, z.B. PEG-Maleimid, PEG-Vinylsulfon, PEG-Iodacetamid sowie PEG-Orthopyridyldisulfid, und liefert dazu Instruktionen für die PEGylierung. Diese PEG-Derivate können erfindungsgemäß unterschiedliche Kettenlängen aufweisen: z.B. sind PEG-Derivate mit Molekulargewichten von 5.000 Dalton, 10.000 Dalton, 20.000 Dalton und 30.000 Dalton kommerziell verfügbar und erfindungsgemäß zu verwenden.

Zur Bestimmung der Protease-Aktivität des mutierten und anschließend PEGylierten Botulinumtoxins wird die Spaltung des den Serotyp entsprechenden SNARE-Proteins quantitativ erfasst. Die Aktivität wird dann ggf. verglichen mit der Aktivität (i) des nativen Neurotoxins (des entsprechenden Serotyps), (ii) des mutierten Neurotoxins mit Tag für die vereinfachte Isolierung und/oder (iii) des mutierten Neurotoxins ohne Tag. Die Aktivität des mutierten und des PEGylierten mutierten Neurotoxins gemäß vorliegender Erfindung ist ähnlich der Aktivität des nativen (nicht-mutierten) Neurotoxins (das heißt, die biologische Aktivität, gemäß vorstehender Definition, des mutierten Neurotoxins und des modifizierten Neurotoxins gemäß der Erfindung ist im Vergleich zu der biologischen Aktivität des nativen Neurotoxins allenfalls geringfügig vermindert im Sinn der oben gegebenen Definition).

Die Gesamtaktivität des PEGylierten mutierten Neurotoxins wird zunächst in einem *ex-vivo-*Modell, dem sog. Zwerchfell- bzw. Hemidiaphragma-Assay bestimmt. Dabei wird die paralysierende Aktivität an einem Nerv-Muskel-Präparat ermittelt. Erfindungsgemäß ist die biologische Aktivität des modifizierten, also des mutierten und anschließend PEGylierten Neurotoxins wenigstens 20 %, vorzugsweise 30-40 % oder 50-70 % oder sogar 75-95 %, der biologischen Aktivität des unmodifizierten (nativen) Proteins (biologische Aktivität ist auch hier gemäß vorstehender Definition zu verstehen).

Die Toxizität des erfindungsgemäß PEGylierten mutierten Neurotoxins kann im LD₅₀-Assay an der Maus geprüft werden, wobei die Dosis bestimmt wird, die nach i.p. Applikation bei der Hälfte einer Gruppe von Mäusen tödlich wirkt:

Die Wirkdauer des erfindungsgemäß PEGylierten mutierten Neurotoxins wird *in vivo,* ebenfalls an der Maus, ermittelt. Nach Injektion einer subletalen Dosis eines PEGylierten mutierten Botulinumtoxins Typ A gemäß vorliegender Erfindung bzw. des entsprechenden nativen Botulinumtoxins in den Gastrocnemius-Muskel der Hinterpfote wird die Zeit bestimmt, in der der Muskel paralysiert bleibt. Die Paralysestärke wird dabei mittels einer Skala klassifiziert. Die Wirkdauer, die bei der Maus kürzer ist als beim Menschen, verlängerte sich je nach dem verwendeten modifizierten Botulinumtoxin Typ A um 30-150 % (gemessen im Vergleich zum un-PEGylierten und unmutierten Botulinumtoxin Typ A).

Das PEGylierte mutierte Botulinumtoxin gemäß vorliegender Erfindung lässt sich in einer geeigneten Formulierung zu einem Fertigarzneimittel verarbeiten, das eine Dosis (oder ein ganzzahliges Vielfaches der Dosis) im Bereich der therapeutischen Dosis (z.B. 100 LD₅₀-. Einheiten pro Injektionsfläschchen) enthält. Gemäß einer bevorzugten Ausführungsform wird die pharmazeutische Zusammensetzung ohne Zusatz von humanem Serumalbumin (HSA) stabilisiert. Sie kann aber auch mit humanem Serumalbumin (HSA) stabilisiert werden. Diesbezüglich besonders bevorzugt ist die Verwendung einer HSA-freien Zusammensetzung zur Stabilisierung von Protein-Wirkstoffen, wie sie in WO 2005/007185 beschrieben ist. Gemäß einer weiteren bevorzugten Ausführungsform liegt die pharmazeutische Zusammensetzung der vorliegenden Erfindung in lyophilisierter Form oder flüssig vor. Beide Formen eignen sich, ggf. nach Aufnahme in einem geeigneten Lösungsmittel, zur i.m. Injektion in den zu behandelnden Muskel.

Das PEGylierte mutierte Botulinumtoxin mit größerer Stabilität und Halbwertzeit bzw. das dieses aufweisende Pharmazeutikum kann eingesetzt werden zur Therapie verschiedener Dystonien, wie spasmodische Dysphonie, Kehlkopfdystonie, cervicale Dystonie, fokale Handdystonie, Blepharospasmus, Strabismus, Zerebralparese, hemifaciale Spasmen, Spastizität, spasmodische Colitis, Anismus, TICS, Tremors, Bruxismus, Analfissur, Achalasie, Dysphagie, Hyperhidrose sowie zur Beseitigung von Gesichtsfalten.

Die nachfolgenden Beispiele erläutern die Erfindung im Detail, ohne sie jedoch einzuschränken auf die dort genannten spezifischen Parameter.

### Beispiele

### Beispiel 1: Klonierung und Expression von Botulinum-Neurotoxin Typ A (BoNT/A)

Zur Klonierung der DNA-Sequenzen der Leichten Kette sowie der Translokationsdomäne wurde aus einer Kultur von *Clostridium botulinum* Typ A (Stamm ATCC 3502) chromosomale DNA isoliert. Durch PCR-Amplifikation mit den Primem SEQ ID NR:1 und SEQ ID NR:2 wurde ein für die Leichte Kette von BoNT/A mit modifizierter Loop-Sequenz codierendes Genfragment erhalten. Das PCR-Amplifikat wurde über die Restriktionsschnittstellen für *Nco* I und *Bgl* II in das Expressionsplasmid pQE-H₁₀ kloniert, welches von pQE-60 abgeleitet wurde und für einen His-Tag (bestehend aus 10 Histidin-Resten) auf der 5'-Seite der Klonierungsstelle codiert. Mit dieser Klonierung wurde das Plasmid pQE-H₁₀-BoNT/A-L generiert. Durch PCR-Amplifikation mit den Primern SEQ ID NR:3 und SEQ ID NR:4 wurde das für die Schwere Kette von BoNT/A codierende Genfragment erhalten. Über die Restriktionsschnittstellen für *Stu* I und *Bgl* II wurde es an das 3'-Ende der Loop-Sequenz der Leichten Kette in pQE-H₁₀-BoNT/A-L kloniert (Plasmid pQE-H₁₀-BoNT/A). Der E. coli-Expressionsstamm M15[pREP4] (Qiagen) wurde mit dem Plasmid pQE-H₁₀-BoNT/A transformiert. Die Expression des rekombinanten Toxins erfolgte durch eine abgestufte Induktion mit 500 µM (Endkonzentration) IPTG bei 25°C über Nacht. Die Zellen wurden in einem 50 mM Phosphatpuffer bei pH 8.0 mit 300 mM NaCl durch Lysozym- und Ultraschallbehandlung aufgeschlossen. Das zentrifugierte Lysat wurde 5 h bei RT inkubiert und nach zwischenzeitlicher Lagerung bei -20°C über eine Ni-NTA-Agarose-Säule chromatographiert. Abschließend erfolgten eine Dialyse der Elutionsfraktionen gegen einen Kopplungspuffer (100 mM Natriumdihydrogenphosphat pH 7.5, 150 mM NaCl, 10 mM EDTA) und eine Bestimmung der Proteinkonzentration Eine Analyse im SDS-Polyacrylamidgel ergab, dass durch Coomassie unter reduzierenden Bedingungen zwei kräftige Banden bei ca. 50 kD und 100 kD sowie eine schwache Bande bei 150 kD angefärbt wurden, während unter nicht-reduzierenden Bedingungen nur eine Bande bei ca. 150 kD zu beobachten war, die dem Bandenmuster eines Botulinum Neurotoxins Typ A in seiner überwiegend zweikettigen, disulfidverbrückten Struktur entspricht.

### Beispiel 2: Klonierung und Expression von Botulinum-Neurotoxin Typ B (BoNT/B)

Klonierung und Expression eines N-terminal mit einem Histidin-Tag (bestehend aus 10 Histidin-Resten) versehenen Botulinum Neurotoxins Typ B erfolgte analog zum Typ-A-Toxin. Zur Amplifikation der Leichten und Schweren Kette wurden chromosomale DNA aus *Clostridium botulinum* Typ B (Stamm Okra) sowie die Primer SEQ ID NR:5 und SEQ ID NR:6 bzw. SEQ ID NR:7 und SEQ ID NR:8 eingesetzt.

### Beispiel 3: Klonierung und Expression von Botulinum-Neurotoxin Typ C1 (BoNT/C1)

Klonierung und Expression eines N-terminal mit einem His-Tag (bestehend aus 10 Histidin-Resten) versehenen Botulinum Neurotoxins Typ C1 erfolgte analog zum Typ-A-Toxin. Zur Amplifikation der Leichten und Schweren Kette wurden chromosomale DNA aus *Clostridium botulinum* Typ C (Stamm 205) sowie die Primer SEQ ID NR:9 und SEQ ID NR:10 bzw. SEQ ID NR:11 und SEQ ID NR:12 eingesetzt.

### Beispiel 4: Klonierung und Expression von C-H₁₀-BoNT/A

Um eine N-terminale PEGylierung zu ermöglichen, wurde N-terminal vom Histidin-Tag (bestehend aus 10 Histidin-Resten) ein Cystein-Rest an die Aminosäure-Sequenz angehängt. Dies wurde durch gerichtete Mutagenese im Sequenzbereich von pQE-H₁₀-BoNT/A, der für den His-Tag codiert, erreicht. Hierfür wurde der QuickChange Site Directed Mugenesis Kit der Firma Stratagene eingesetzt. Die Mutagenesereaktion erfolgte mit den Primern SEQ ID NR:13 und SEQ ID NR:14. Der Nucleotidaustausch in der DNA-Sequenz wurde durch DNA-Sequenzierung der isolierten Klone verifiziert.. Die Expression und Reinigung des mutierten Toxins erfolgten analog zu Beispiel 1.

### Beispiel 5: PEGylierung von C-H₁₀-BoNT/A

1.2 mg C-H₁₀-BoNT/A wurden zur Reduktion disulfidverbrückter Dimere 30 Minuten mit 1 mM DTT inkubiert. Zur Abtrennung des Reduktionsmittels wurde über eine PD-10-Säule auf Kopplungspuffer umgepuffert. Die Toxinlösung wurde mittels Ultrafiltration auf 3.6 mg/ml konzentriert. Ein kleiner Teil wurde als Kontrollprobe unbehandelt inkubiert, die restliche Lösung mit einem 5 fachen molaren Überschuss mPEG-Mal-5000 (Nektar Therapeutics) versetzt und über Nacht bei Umgebungstemperatur rotiert. Um mögliche Derivatisierungsreaktionen an weiteren Cystein-Resten während der Probenvorbereitung für die SDS-PAGE zu vermeiden, wurde das PEGylierungsreagenz mit einem 5 fachen Überschuss L-Cystein abgesättigt. Im SDS-Gel zeigte sich im Vergleich zum Kontrollansatz bei nicht-reduzierendem Auftrag eine starke zusätzliche Bande mit leicht verringerter Mobilität, während die Intensität der ursprünglichen Toxin-Bande bei 150 kD deutlich vermindert war (Fig. 2).

### Beispiel 6: In vitro-Aktivitätstest

Die Bestimmung der spezifischen Protease-Aktivität (das heißt, der katalytischen Aktivität ohne Bindung bzw. Translokation) der PEGylierten mutierten BoNT/A-Derivate erfolgte im ELISA-Format. Hierfür wurde ein rekombinantes Polypeptid kloniert, das im N-terminalen Bereich aus dem gebräuchlichen Fusionspartner Glutathion-S-Transferase (GST) und einer C-terminalen Peptidsequenz zusammengesetzt ist, die die C-terminalen 17 Aminosäure-Reste von SNAP-25 umfasst. Diese 17 Aminosäure-Reste repräsentieren den Bereich des Substratproteins SNAP-25, in dem BoNT/A spezifisch spaltet. Nach Beschichtung einer Mikrotiterplatte mit dem Fusionsprotein wurde mit H₁₀-BoNT/A als Referenzprobe bzw. mit den Mutanten in PEGylierter und un-PEGylierter Form inkubiert. Die Detektion der jeweils generierten Spaltprodukte erfolgte mit einem Antikörper, der den neu entstandenen C-Terminus spezifisch erkennt. Die Werte für C-H₁₀-BoNT/A in ihrer un-PEGylierten sowie PEGylierten Form sowie für H₁₀-BoNT/A (Referenzprobe) sind in Tabelle 3 aufgeführt. Die Schwankungsbreite des Assays berücksichtigend läßt sich feststellen, dass durch die Einführung der Mutation und anschließende PEGylierung die katalytische Aktivität des Botulinumtoxins nicht vermindert, sondern eher noch gesteigert wurde.

**Tabelle 3**

| | **Relative Aktivität [%]** |
|---|---|
| H₁₀-BoNT/A | 100 |
| C-H₁₀-BoNT/A | 100 |
| mPEG-C-H₁₀-BoNT/A | 121,1 |

Die spezifische Aktivität wurde in Protease-Einheiten/ng Protein bestimmt.

### Beispiel 7: Bestimmung der Aktivität ex vivo im Hemidiaphragma-Assay

Zur Bestimmung der Gesamtaktivität der Toxin-Derivate, d.h. der Bindung der modifizierten Neurotoxine an den Rezeptor der Zielzellen und der Translokation in die Nervenzelle und Proteolyse des SNARE-Substrats wurde die Paralysezeit eines Nerv-Muskel-Präparates aus Maus nach Intoxikation ermittelt. Als Referenzprobe diente wiederum H₁₀-BoNT/A. Die Werte der relativen Aktivitäten sind in Tabelle 4 aufgeführt. Die Verringerung der Aktivität der modifizierten Botulinumtoxine gemäß vorliegender Erfindung gegenüber der Referenzprobe auf 20-30 % beruht also offensichtlich auf einer verringerten Fähigkeit der Bindung des Toxins an die Zielzellen und der verringerten Fähigkeit, das Toxin in die Zielzellen zu translozieren.

**Tabelle 4**

| | **relative Aktivität [%]** |
|---|---|
| H₁₀-BoNT/A | 100 |
| C-H₁₀-BoNT/A | 22,5 |
| mPEG-C-H₁₀-BoNT/A | 30 |

### Beispiel 8: Wirkdauer von PEGyliertem Botulinumtoxin Typ A

Die Wirkdauer von PEGyliertem mutierten Botulinumtoxin (mPEG-C-H₁₀-BoNT/A) wurde an CD 1-Mäusen getestet. Jeweils 10 Mäuse erhielten i.m. Injektionen (2 x 0.05 mL) von (i) mutiertem, (ii) PEGyliertem mutiertem bzw. (iii) nativem Botulinumtoxin in einer Dosierung von je 0.4 oder 0.6 LD₅₀-Units/Maus (in physiologischer Kochsalzlösung + 1 mg/mL HSA) in den Gastrocnemius-Muskel der Hinterpfote. Anschließend wurde täglich die Paralyse des Muskels anhand einer Skala (minimale, leichte, schwere Paralyse) beurteilt. Nach 25 Tagen war bei den Tieren, die mit nativem Neurotoxin behandelt worden waren, keine Paralyse des Muskels mehr zu beobachten. Bei den Tieren, die mit mutiertem bzw. PEGyliertem mutierten Botulinumtoxin behandelt worden waren, war die Wirkdauer um 7-20 Tage verlängert.

## Patentansprüche

1. Modifiziertes Botulinumtoxin, das eine natürliche Schwere Kette und eine modifizierte Leichte Kette aufweist, **dadurch gekennzeichnet, dass** die Modifikation der Leichten Kette darin besteht, dass
(a) (i) sie an ihrem N-Terminus eine Verlängerung der Kette aufweist, die in der Richtung vom N- zum C-terminalen Ende die Struktur -(C)ₙ-(tag)ₘ (X)ₖ- hat, und (ii) wenigstens einer der Cystein-Reste in der Verlängerung der Kette an wenigstens eine Kette PEG gekoppelt ist, oder dass
(b) mindestens ein Aminosäure-Rest, und maximal 20 Aminosäure-Reste, der natürlicherweise an ihrem N-Terminus sitzenden Aminosäure-Reste zu einem Cystein-Rest mutiert ist/sind, wobei wenigstens einer der maximal 20 Cystein-Reste am N-Terminus an wenigstens eine Kette PEG gekoppelt ist, und die Leichte Kette ggf. zusätzlich eine N-terminale Verlängerung aufweist, so dass die Sequenz der Leichten Kette in der Richtung vom N- zum C-terminalen Ende die Struktur -(tag)ₘ-(X)ₖ-BoNT(X1-20C) hat,
wobei
C für einen Cystein-Rest,
tag für einen beliebigen Tag und
X für den Rest einer beliebigen natürlich vorkommenden Aminosäure stehen;
wobei
n eine ganze Zahl von 1 bis 50 ist;
wobei
m = 0 oder m = 1 und
k = 0 oder k eine ganze Zahl von 1 bis 50 ist.

2. Das Botulimuntoxin nach Anspruch 1, **dadurch gekennzeichnet, dass** keiner der natürlicherweise in der Schweren und Leichten Kette von Botulinumtoxin vorkommenden Cystein-Reste PEGyliert ist.

3. Das Botulinumtoxin nach Anspruch 1 oder 2, **dadurch gekennzeichnet, dass** der Tag ein His-Tag oder ein Strep-Tag ist.

4. Das Botulinumtoxin nach einem der Ansprüche 1 bis 3, **dadurch gekennzeichnet, dass** das Botulinumtoxin ein Botulinumtoxin der Typen A, B, C1, D, E, F oder G ist und eine der folgenden Bedingungen gilt:
(a) n = 1, 2 oder 3, m = 0 oder 1, k = 0 oder k ≠0,
(b1) n = 1, m = 1, k = 0; (b2) n = 2, m = 1, k = 0; (b3) n = 3, m = 1, k = 0;
(c1) n = 1, m = 0, k = 0; (c2) n = 2, m = 0, k = 0; (c3) n = 3, m = 0, k = 0;
(d1) n = 1, m = 1, k ≠0; (d2) n = 2, m = 1, k ≠0; (d3) n = 3, m = 1, k ≠0;
(e1) n = 1, m = 0, k ≠0; (e2) n = 2, m = 0, k ≠0; (e3) n = 3, m = 0, k ≠0,
wobei die Toxine pro Molekül an eine, an zwei oder an drei PEG-Moleküle gekoppelt sind, je nachdem, ob n = 1, 2 oder 3.

5. Das Botulinumtoxin nach einem der Ansprüche 1 bis 4, **dadurch gekennzeichnet, dass** die Verlängerung der Leichten Kette eine der folgenden Sequenzen hat:
-(C)₁-(tag)₁-(X)₀-, -(C)₂-(tag)₁-(X)₀-, -(C)₃-(tag)₁-(X)₀-, -(C)₄-(tag)₁-(X)₀-, -(C)₅-(tag)₁-(X)₀-, -(C)₁-(tag)₁-(X)₁-. -(C)₂-(tag)₁-(X)₁-, -(C)₃-(tag)₁-(X)₁-, -(C)₄-(tag)₁-(X)₁-, -(C)₅-(tag)₁-(X)₁-, -(C)₁-(tag)₁-(X)₂-, -(C)₂-(tag)₁-(X)₂-, -(C)₃-(tag)₁-(X)₂-, -(C)₄-(tag)₁-(X)₂-, -(C)₅-(tag)₁-(X)₂-, -(C)₁-(tag)₁-(X)₃-, -(C)₂-(tag)₁-(X)₃-, -(C)₃-(tag)₁-(X)₃-, -(C)₄-(tag)₁-(X)₃-, -(C)₅-(tag)₁-(X)₃-, -(C)₁-(tag)₁-(X)₄-, -(C)₂-(tag)₁-(X)₄-, -(C)₃-(tag)₁-(X)₄-, -(C)₄-(tag)₁-(X)₄-,-(C)₅-(tag)₁-(X)₄-,
wobei für jede dieser 25 Sequenzen m auch 0 sein kann, und/oder dass jeweils alle in der Verlängerung der Leichten Kette vorkommenden Cystein-Reste auch PEGyliert sind.

6. Das Botulinumtoxin nach einem der Ansprüche 1 bis 3, **dadurch gekennzeichnet, dass** das Botulinumtoxin ein Botulinumtoxin der Typen A, B, C1, D, E, F oder G ist und eine der folgenden Bedingungen gilt:
(a) m = 1, k = 0, nur einer der 20 N-terminalen Aminosäure-Reste ist durch einen Cystein-Rest ersetzt, insbesondere nur der Rest an Position 1, 2, 3, 4, 5, 6, 7, 8, 9, oder 10;
(b) m = 0, k = 0, nur einer der 20 N-terminalen Aminosäure-Reste ist durch einen Cystein-Rest ersetzt, insbesondere nur der Rest an Position 1, 2, 3, 4, 5, 6, 7, 8, 9, oder 10;
(c) m = 1, k ≠0, nur einer der 20 N-terminalen Aminosäure-Reste ist durch einen Cystein-Rest ersetzt, insbesondere nur der Rest an Position 1, 2, 3, 4, 5, 6, 7, 8, 9, oder 10;
(d) m = 0, k ≠0, nur einer der 20 N-terminalen Aminosäure-Reste ist durch einen Cystein-Rest ersetzt, insbesondere nur der Rest an Position 1, 2, 3, 4, 5, 6, 7, 8, 9, oder 10;
(e) m = 1, k = 0, nur zwei der 20 N-terminalen Aminosäure-Reste sind durch einen Cystein-Rest ersetzt, insbesondere nur die Reste an den Positionen 1 und 3, 1 und 4, 2 und 4, 1 und 5, 2 und 5, 3 und 5, 1 und 6, 2 und 6, 3 und 6 oder 4 und 6;
(f) m = 0, k = 0, nur zwei der 20 N-terminalen Aminosäure-Reste sind durch einen Cystein-Rest ersetzt, insbesondere nur die Reste an den Positionen 1 und 3, 1 und 4, 2 und 4, 1 und 5, 2 und 5, 3 und 5, 1 und 6, 2 und 6, 3 und 6 oder 4 und 6;
(g) m = 1, k ≠0, nur zwei der 20 N-terminalen Aminosäure-Reste sind durch einen Cystein-Rest ersetzt, insbesondere nur die Reste an den Positionen 1 und 3, 1 und 4, 2 und 4, 1 und 5, 2 und 5, 3 und 5, 1 und 6, 2 und 6, 3 und 6 oder 4 und 6;
(h) m = 0, k ≠0, nur zwei der 20 N-terminalen Aminosäure-Reste sind durch einen Cystein-Rest ersetzt, insbesondere nur die Reste an den Positionen 1 und 3, 1 und 4, 2 und 4, 1 und 5, 2 und 5, 3 und 5, 1 und 6, 2 und 6, 3 und 6 oder 4 und 6;
(i) m = 1, k = 0, nur zwei der 20 N-terminalen Aminosäure-Reste sind durch einen Cystein-Rest ersetzt, insbesondere nur die Reste an den Positionen 1 und 2, 2 und 3, 3 und 4, 4 und 5 oder 5 und 6;
(j) m = 0, k = 0, nur zwei der 20 N-terminalen Aminosäure-Reste sind durch einen Cystein-Rest ersetzt, insbesondere nur die Reste an den Positionen 1 und 2, 2 und 3, 3 und 4, 4 und 5 oder 5 und 6;
(k) m = 1, k ≠0, nur zwei der 20 N-terminalen Aminosäure-Reste sind durch einen Cystein-Rest ersetzt, insbesondere nur die Reste an den Positionen 1 und 2, 2 und 3, 3 und 4, 4 und 5 oder 5 und 6;
(l) m = 0, k ≠0, nur zwei der 20 N-terminalen Aminosäure-Reste sind durch einen Cystein-Rest ersetzt, insbesondere nur die Reste an den Positionen 1 und 2, 2 und 3, 3 und 4, 4 und 5 oder 5 und 6,
wobei die Toxine pro Molekül an ein oder an zwei PEG-Moleküle gekoppelt sind, je nachdem, ob nur ein oder zwei Cystein-Reste N-terminal eingeführt wurden.

7. Das Botulinumtoxin nach einem der Ansprüche 1 bis 3 und 6, **dadurch gekennzeichnet, dass** das modifizierte Botulinumtoxin ein Botulinumtoxin ist, dessen Leichte Kette so modifiziert ist, dass diese an ihrem N-Terminus eine Verlängerung der Kette aufweist, wobei der N-Terminus der verlängerten Kette eine der folgenden Sequenzen aufweist:
(a) -(tag)₁-(X)₀-BoNT(P1C), -(tag)₁-(X)₀-BoNT(F2C), -(tag)₁-(X)₀-BoNT(V3C), -(tag)₁-(X)₀-BoNT(N4C), (tag)₁-(X)₀-BoNT(K5C),
(b) -(tag)₁-(X)₁-BoNT(P1C), -(tag)₁-(X)₁-BoNT(F2C), -(tag)₁-(X)₁-BoNT(V3C), -(tag)₁-(X)₁-BoNT(N4C), -(tag)₁-(X)₁-BoNT(K5C),
(c) -(tag)₁-(X)₂-BoNT(P1C), -(tag)₁-(X)₂-BoNT(F2C), -(tag)₁-(X)₂-BONT(V3C), -(tag)₁-(X)₂-BoNT(N4C), -(tag)₁-(X)₂-BoNT(K5C),
(d) -(tag)₁-(X)₃-BoNT(P1C), -(tag)₁-(X)₃-BoNT(F2C), -(tag)₁-(X)₃-BoNT(V3C), -(tag)₁-(X)₃-BoNT(N4C), -(tag)₁-(X)₃-BoNT(K5C),
(e) -(tag)₁-(X)₄-BoNT(P1C), -(tag)₁-(X)₄-BoNT(F2C), -(tag)₁-(X)₄-BoNT(V3C), -(tag)₁-(X)₄-BoNT(N4C), -(tag)₁-(X)₄-BoNT(K5C),
wobei für jede der vorstehend aufgelisteten 25 bevorzugten Ausführungsformen (tag)₁auch-(tag)₀ sein kann, und/oder jeweils alle N-terminal eingeführten Cystein-Reste auch PEGyliert sind.

8. Das Botulinumtoxin nach einem der vorhergehenden Ansprüche, **dadurch gekennzeichnet, dass** das Botulinumtoxin ein Botulinumtoxin vom Typ A, B oder C1 ist.

9. Das Botulinumtoxin nach einem der vorhergehenden Ansprüche, **dadurch gekennzeichnet, dass** das modifizierte Botulinumtoxin wenigstens 20 %, vorzugsweise 30-40 %, 50-70 % oder 75-95 % der biologischen Aktivität des entsprechenden natürlichen (unmodifizierten, nativen) Botulinumtoxins und eine gegenüber diesem erhöhte Stabilität aufweist.

10. Das Botulinumtoxin nach einem der vorhergehenden Ansprüche, **dadurch gekennzeichnet, dass** die modifizierte Leichte Kette *in vivo* in das Cytosol der Motoneuronen transloziert wird.

11. Das Botulinumtoxin nach einem der vorhergehenden Ansprüche, **dadurch gekennzeichnet, dass** die modifizierte Leichte Kette im Cytosol der Motoneuronen eine höhere Stabilität aufweist als das entsprechende native Botulinumtoxin.

12. Pharmazeutische Zusammensetzung, wobei die Zusammensetzung das modifizierte Botulinumtoxin nach einem der vorhergehenden Ansprüche umfasst.

13. Die pharmazeutische Zusammensetzung nach Anspruch 12, wobei die Zusammensetzung ohne Zusatz von humanem Serumalbumin (HSA) stabilisiert wird.

14. Die pharmazeutische Zusammensetzung nach Anspruch 12 oder 13, wobei die Zusammensetzung in lyophilisierter Form oder flüssig vorliegt und wobei sich beide Formen, ggf. nach Aufnahme in einem geeigneten Lösungsmittel, zur i.m. Injektion eignen.

15. Die pharmazeutische Zusammensetzung nach Anspruch 12, wobei die Zusammensetzung für eine Verwendung zur Therapie von Dystonien sowie Beseitigung von Gesichtsfalten vorgesehen ist.

## Claims

1. Modified botulinum toxin exhibiting a natural heavy-chain and a modified light-chain **characterized in that** the modification of the light-chain resides **in that**
(a) (i) it exhibits an elongation of the chain at its N-terminus, wherein the elongation possesses the structure -(C)ₙ-(tag)ₘ-(X)ₖ- in the direction from the N- to the C-terminal end, and (ii) at least one of the cysteine residues in the elongation of the chain is coupled to at least one chain PEG, or that
(b) at least one amino acid residue, and maximally 20 amino acid residues of the naturally at its N-terminus located amino acid residues is/are mutated to a cysteine residue,
wherein at least one of the maximally 20 cysteine residues at the N-terminus is coupled to at least one chain of PEG, and optionally the light-chain additionally exhibits a N-terminal elongation such that the sequence of the light-chain possesses the structure - (tag)ₘ-(X)ₖ-BoNT(X1-20C) in the direction from the N- to the C-terminal end,
wherein
C is a cysteine residue,
tag is any tag and
X is the residue of any naturally occurring amino acid;
wherein
n is an integer from 1 to 50;
wherein
m = 0 or m = 1 and
k = 0 or k is an integer from 1 to 50.

2. Botulinum toxin according to claim 1 **characterized in that** none of the cysteine residues naturally occurring in the heavy- and light-chain of botulinum toxin is PEGylated.

3. Botulinum toxin according to claim 1 or 2 **characterized in that** the tag is a his-tag or a strep-tag.

4. Botulinum toxin according to any one of claims 1 to 3 **characterized in that** the botulinum toxin is a botulinum toxin of the types A, B, C1, D, E, F or G and one of the following conditions is fulfilled:
(a) n=1,2 or 3,m=0 or 1,k=0 or k=0,
(b1) n=1,m=1,k=0;(b2)n=2,m=1,k=0;(b3)n=3,m=1,k=0;
(c1)n=1,m=0,k=0;(c2)n=2,m=0,k=0;(c3)n=3,m=0,k=0;
(d1)n=1,m=1,k ≠0;(d2)n-2,m=1,k≠0;(d3)n=3,m=1,k=0;
(e1)n=1,m=0,k ≠0;(e2)n=2,m=0,k ≠0;(e3)n=3,m=0,k =0,
wherein the toxins are coupled to one, two or three PEG molecules per molecule, depending on whether n = 1, 2 or 3.

5. Botulinum toxin according to any one of claims 1 to 4 **characterized in that** the elongation of the light-chain exhibit one of the following sequences:
-(C)₁-(tag)₁-(X)₀-,-(C)₂-(tag)₁-(X)₀-,-(C)₃-(tag)₁-(X)₀-,-(C)4-(tag)₁-(X)₀-, -(C)₅-(tag)₁-(X)₀-, -(C)₁-(tag)₁-(X)₁-, -(C)₂-(tag)₁-(X)₁-, -(C)₃-(tag)₁-(x)₁-, -(C)₄-(tag)₁-(X)₁-, -(C)₅-(tag)₁-(X)₁-, -(C)₁-(tag)₁-(X)₂-, -(C)₂-(tag)₁-(X)2-, -(C)₃-(tag)₁-(X)₂-, -(C)₄-(tag)₁-(X)₂-, -(C)₅-(tag)₁-(X)₂-, -(C)₁-(tag)₁-(X)₃-, -(C)₂-(tag)₁-(X)₃-, -(C)₃-(tag)₁-(X)₃-, -(C)₄-(tag)₁-(X)₃-, -(C)₅-(tag)₁-(X)₃-, -(C)₁-(tag)₁-(X)₄-, -(C)₂-(tag)₁-(X)₄-, -(C)₃-(tag)₁-(X)₄-, -(C)₄-(tag)₁-(X)₄-,-(C)₅-(tag)₁-(X)₄-,
wherein for each of these 25 sequences m may also be 0, and/or that respectively each of the cysteine residues occurring in the elongation of the light-chain is also PEGylated.

6. Botulinum toxin according to any one of claims 1 to 3 **characterized in that** the botulinum toxin is a botulinum toxin of the types A, B, C1, D, E, F or G and one of the following conditions is fulfilled:
(a) m = 1, k = 0, only one of the 20 N-terminal amino acid residues is replaced by a cysteine residue, particularly only the residue at position 1, 2, 3, 9, 5, 6, 7, 8, 9, or 10;
(b) m = 0, k = 0, only one of the 20 N-terminal amino acid residues is replaced by a cysteine residue, particularly only the residue at position 1, 2, 3, 4, 5, 6, 7, 8, 9, or 10;
(c) m = 1, k ≠0, only one of the 20 N-terminal amino acid residues is replaced by a cysteine residue, particularly only the residue at position 1, 2, 3, 4, 5, 6, 7, 8, 9, or 10;
(d) m = 0, k ≠0, only one of the 20 N-terminal amino acid residues is replaced by a cysteine residue, particularly only the residue at position 1, 2, 3, 4, 5, 6, 7, 8, 9, or 10;
(e) m = 1, k = 0, only two of the 20 N-terminal amino acid residues are replaced by a cysteine residue, particularly only the residues at the positions 1 and 3, 1 and 4, 2 and 4, 1 and 5, 2 and 5, 3 and 5, 1 and 6, 2 and 6, 3 and 6 or 4 and 6;
(f) m = 0, k = 0 only two of the 20 N-terminal amino acid residues are replaced by a cysteine residue, particularly only the residues at the positions 1 and 3, 1 and 4, 2 and 4, 1 and 5, 2 and 5, 3 and 5, 1 and 6, 2 and 6, 3 and 6 or 4 and 6;
(g) m = 1, k ≠0, only two of the 20 N-terminal amino acid residues are replaced by a cysteine residue, particularly only the residues at the positions 1 and 3, 1 and 4, 2 and 4, 1 and 5, 2 and 5, 3 and 5, 1 and 6, 2 and 6, 3 and 6 or 4 and 6;
(h) m = 0, k≠0, only two of the 20 N-tenninal amino acid residues are replaced by a cysteine residue, particularly only the residues at the positions 1 and 3, 1 and 4, 2 and 4, 1 and 5, 2 and 5, 3 and 5, 1 and 6, 2 and 6, 3 and 6 or 4 and 6;
(i) m = 1, k = 0, only two of the 20 N-terminal amino acid residues are replaced by a cysteine residue, particularly only the residues at the positions 1 and 2, 2 and 3, 3 and 4, 4 and 5 or 5 and 6;
(j) m = 0, k = 0, only two of the 20 N-terminal amino acid residues are replaced by a cysteine residue, particularly only the residues at the positions 1 and 2, 2 and 3, 3 and 4, 4 and 5 or 5 and 6;
(k) m = 1, k #0, only two of the 20 N-terminal amino acid residues are replaced by a cysteine residue, particularly only the residues at the positions 1 and 2, 2 and 3, 3 and 4, 4 and 5 or 5 and 6;
(1) m = 0, k ≠0, only two of the 20 N-terminal amino acid residues are replaced by a cysteine residue, particularly only the residues at the positions 1 and 2, 2 and 3, 3 and 4, 4 and 5 or 5 and 6,
wherein the toxins are coupled to one or two PEG molecules per molecule, depending on whether only one or two cysteine residues are introduced N-terminally.

7. Botulinum toxin according to any one of claims 1 to 3 and 6 **characterized in that** the modified botulinum toxin is a botulinum toxin, wherein the light-chain of the botulinum toxin is modified such that its N-terminus exhibits an elongation of the chain, wherein the N-terminus of the elongated chain exhibits one of the following sequences:
(a) -(tag)₁-(X)₀-BoNT(P1C), -(tag)₁-(X)₀-BONT(F2C), -(tag)₁-(X)₀-BoNT(V3C), -(tag)₁-(X)₀-BoNT(N4C), (tag)₁-(X)₀-BoNT(K5C),
(b) -(tag)₁-(X)₁-BoNT(P1C), -(tag)₁-(X)₁-BoNT(F2C), -(tag)₁-(X)₁-BoNT(V3C), -(tag)₁-(X)₁-BoNT(N4C), -(tag)₁-(X)₁-BoNT(K5C),
(c) -(tag)₁-(X)₂-BoNT(P1C), -(tag)₁-(X)₂-BoNT(F2C), -(tag)₁-(X)₂-BoNT(V3C), -(tag)₁-(X)₂-BoNT(N4C), -(tag)₁-(X)₂-BoNT(K5C),
(d) -(tag)₁-(X)₃-BoNT(P1C), -(tag)₁-(X)₃-BoNT(F2C), -(tag)₁-(X)₃-BoNT(V3C), -(tag)₁-(X)₃-BoNT(N4C), -(tag)₁-(X)₃-BoNT(K5C),
(e) -(tag)₁-(X)₄-BoNT(P1C), -(tag)₁-(X)₄-BoNT(F2C), -(tag)₁-(X)₄-BoNT(V3C), -(tag)₁-(X)₄-BoNT(N4C), -(tag)₁-(X)₄-BoNT(K5C),
wherein for each of the 25 previously listed preferred embodiments (tag)₁ may also be (tag)₀, and/or respectively all N-terminally introduced cysteine residues are also PEGylated.

8. Botulinum toxin according to any one of the preceding claims **characterized in that** the botulinum toxin is botulinum toxin of the type A, B or C1.

9. Botulinum toxin according to any of the preceding claims, **characterized in that** the modified botulinum toxin exhibits at least 20%, preferably 30-40%, 50-70% or 75-95% of the biological activity of the respective natural (unmodified, native) Botulinum toxin and exhibits increased stability compared to the natural (unmodified, native) Botulinum toxin.

10. Botulinum toxin according to any one of the preceding claims **characterized in that** the modified light-chain is translocated *in vivo* into the cytosol of the motoneurons.

11. Botulinum toxin according to any one of the preceding claims, **characterized in that** the modified light-chain exhibits a higher stability in the cytosol of the motoneurons compared to the respective native botulinum toxin.

12. Pharmaceutical composition, wherein the composition comprises the modified botulinum toxin according to any one of the preceding claims.

13. Pharmaceutical composition according to claim 12, wherein the composition is stabilized without the addition of human serum albumin (HSA).

14. Pharmaceutical composition according to claim 12 or 13, wherein the composition is lyophilized or liquid and wherein both forms are suitable for an i.m. injection optionally after dissolving it in a suitable solvent.

15. Pharmaceutical composition according to claim 12, wherein the composition is for the therapy of dystonias as well as for the removal of face wrinkles.

## Revendications

1. Toxine botulique modifiée, comprenant une chaîne lourde naturelle et une chaîne légère modifiée, **caractérisée en ce que** la modification de la chaîne légère consiste **en ce que** :
(a) (i) elle présente en position N-terminale un prolongement de chaîne qui a la structure -(C)ₙ-(tag)ₘ-(X)ₖ- dans la direction allant de son extrémité N-terminale à son extrémité C-terminale, et (ii) au moins un des résidus de cystéine dans le prolongement de la chaîne est couplé à au moins une chaîne PEG, ou que
(b) au moins un résidu d'acide aminé, et au maximum 20 résidus d'acides aminés, des résidus d'acides aminés se trouvant naturellement sur l'extrémité N-terminale est/sont mutés en un résidu de cystéine, où au moins un des au maximum 20 résidus de cystéine sur l'extrémité N-terminale est couplé à au moins une chaîne PEG, et la chaîne légère présente le cas échéant en plus un prolongement N-terminal tel que la séquence de la chaîne légère a la structure -(tag)ₘ-(X)ₖ-BoNT(Xl-20C) dans la direction allant de son extrémité N-terminale à son extrémité C-terminale,
où
C représente un résidu de cystéine,
tag représente un tag quelconque et
X représente le résidu d'un acide aminé quelconque existant naturellement ;
où
n représente un nombre entier entre 1 et 50 ;
où
m=0 ou m=1 et
k =0 ou k représente un nombre entier de 1 à 50.

2. Toxine botulique selon la revendication 1, **caractérisée en ce qu'**aucun des résidus de cystéine existant naturellement dans les chaînes lourde et légère de la toxine botulique n'est PEGylé.

3. Toxine botulique selon la revendication 1 ou 2, **caractérisée en ce que** le tag est un tag His ou un tag Strep.

4. Toxine botulique selon l'une des revendications 1 à 3, **caractérisée en ce que** la toxine botulique est une toxine botulique de type A, B, C1, D, E, F ou G et l'une des conditions suivantes est remplie :
(a)n=1,2 ou 3,m=0 ou 1,k=0 ou k≠0,
(b1)n=1,m=1,k=0; (b2)n=2,m=1,k=0; (b3)n=3,m=1,k=0;
(c1)n=1,m=0,k=0; (c2)n=2,m=0,k=0; (c3)n=3,m=0,k=0;
(d1)n=1,m=1,k≠0; (d2)n=2,m=1,k≠0; (d3)n=3,m=1,k≠0;
(e1)n=1,m=0,k≠0; (e2)n=2,m=0,k≠0; (e3)n=3,m=0,k≠0;
où les toxines sont couplées à une, à deux ou à trois molécules de PEG par molécule, selon que n=1,2 ou 3.

5. Toxine botulique selon l'une des revendications 1 à 4, **caractérisée en ce que** le prolongement de la chaîne légère a l'une des séquences suivantes :
-(C)₁-(tag)₁-(X)₀-, -(C)₂-(tag)₁-(X)₀-, -(C)₃-(tag)₁-(X)₀-, -(C)₄-(tag)₁-(X)₀-, -(C)₅-(tag)₁-(X)₀-, -(C)₁-(tag)₁-(X)₁-, -(C)₂-(tag)₁-(X)₁-, -(C)₃-(tag)-(X)₁-, -(C)₄-(tag)₁-(X)₁-, -(C)₅-(tag)₁-(X)₁-, -(C)₁-(tag)₁-(X)₂-, -(C)₂-(tag)₁-(X)₂-, -(C)₃-(tag)₁-(X)₂-, -(C)₄-(tag)₁-(X)₂-, -(C)₅-(tag)₁-(X)₂-, -(C)₁-(tag)₁-(X)₃-, -(C)₂-(tag)₁-(X)₃-, -(C)₃-(tag)₁-(X)₃-, -(C)₄-(tag)₁-(X)₃-, -(C)₅-(tag)₁-(X)₃-, -(C)₁-(tag)₁-(X)₄-, -(C)₂-(tag)₁-(X)₄-, -(C)₃-(tag)₁-(X)₄-, -(C)₄-(tag)₁-(X)₄-, -(C)₅-(tag)₁-(X)₄-,
où, pour chacune de ces 25 séquences, m peut être aussi égal à 0, et/ou **en ce que** respectivement tous les résidus de cystéine existant dans le prolongement de la chaîne légère sont aussi PEGylés.

6. Toxine botulique selon l'une des revendications 1 à 3, **caractérisée en ce que** la toxine botulique est une toxine botulique de type A, B, C1, D, E, F ou G et l'une des conditions suivantes est remplie :
(a) m = 1, k = 0, seulement un des 20 résidus d'acides aminés N-terminaux est remplacé par un résidu de cystéine, en particulier seulement le résidu en position 1, 2, 3, 4, 5, 6, 7, 8, 9 ou 10;
(b) m = 0, k = 0, seulement un des 20 résidus d'acides aminés N-terminaux est remplacé par un résidu de cystéine, en particulier seulement le résidu en position 1, 2, 3, 4, 5, 6, 7, 8, 9 ou 10 ;
(c) m = 1, k≠0, seulement un des 20 résidus d'acides aminés N-terminaux est remplacé par un résidu de cystéine, en particulier seulement le résidu en position 1, 2, 3, 4, 5, 6, 7, 8, 9 ou 10;
(d) m = 0, k≠0, seulement un des 20 résidus d'acides aminés N-terminaux est remplacé par un résidu de cystéine, en particulier seulement le résidu en position 1, 2, 3, 4, 5, 6, 7, 8, 9 ou 10;
(e) m = 1, k = 0, seulement deux des 20 résidus d'acides aminés N-terminaux sont remplacés par un résidu de cystéine, en particulier seulement les résidus aux positions 1 et 3, 1 et 4, 2 et 4, 1 et 5,2 et 5,3 et 5,1 et 6,2 et 6,3 et 6 ou 4 et 6;
(f) m = 0, k = 0, seulement deux des 20 résidus d'acides aminés N-terminaux sont remplacés par un résidu de cystéine, en particulier seulement les résidus aux positions 1 et 3,1 et 4,2 et 4,1 et 5,2 et 5,3 et 5,1 et 6,2 et 6,3 et 6 ou 4 et 6;
(g) m = 1, k≠ 0, seulement deux des 20 résidus d'acides aminés N-terminaux sont remplacés par un résidu de cystéine, en particulier seulement les résidus aux positions 1 et 3,1 et 4,2 et 4,1 et 5,2 et 5,3 et 5,1 et 6,2 et 6,3 et 6 ou 4 et 6;
(h) m = 0, k ≠ 0, seulement deux des 20 résidus d'acides aminés N-terminaux sont remplacés par un résidu de cystéine, en particulier seulement les résidus aux positions 1 et 3,1 et 4,2 et 4,1 et 5,2 et 5,3 et 5,1 et 6,2 et 6,3 et 6 ou 4 et 6;
(i) m = 1, k = 0, seulement deux des 20 résidus d'acides aminés N-terminaux sont remplacés par un résidu de cystéine, en particulier seulement les résidus aux positions 1 et 2,2 et 3,3 et 4,4 et 5 ou 5 et 6;
(j) m = 0, k = 0, seulement deux des 20 résidus d'acides aminés N-terminaux sont remplacés par un résidu de cystéine, en particulier seulement les résidus aux positions 1 et 2,2 et 3,3 et 4,4 et 5 ou 5 et 6;
(k) m = 1, k ≠ 0, seulement deux des 20 résidus d'acides aminés N-terminaux sont remplacés par un résidu de cystéine, en particulier seulement les résidus aux positions 1 et 2,2 et 3,3 et 4,4 et 5 ou 5 et 6;
(l) m = 0, k ≠ 0, seulement deux des 20 résidus d'acides aminés N-terminaux sont remplacés par un résidu de cystéine, en particulier seulement les résidus aux positions 1 et 2,2 et 3,3 et 4,4 et 5 ou 5 et 6;
où les toxines sont couplées à une ou à deux molécules de PEG par molécule, selon que seulement un ou deux résidus de cystéine ont été introduits sur l'extrémité N-terminale.

7. Toxine botulique selon l'une des revendications 1 à 3 et 6, **caractérisée en ce que** la toxine botulique modifiée est une toxine botulique, dont la chaîne légère est modifiée afin que celle-ci présente en position N-terminale un prolongement de chaîne, où l'extrémité N-terminale de la chaîne prolongée présente une des séquences suivantes :
(a) -(tag)₁-(X)₀-BoNT(P1C), -(tag)₁-(X)₀-BoNT(F2C), -(tag)₁-(X)₀-BoNT(V3C), -(tag)₁-(X)₀-BoNT(N4C), -(tag)₁-(X)₀-BoNT(K5C),
(b) -(tag)₁-(X)₁-BoNT(P1C), -(tag)-(X)₁-BoNT(F2C), -(tag)₁-(X)₁-BoNT(V3C), -(tag)₁-(X)₁-BoNT(N4C), -(tag)₁-(X)₁-BoNT(K5C),
(c) -(tag)₁-(X)₂-BoNT(P1C), -(tag)₁-(X)₂-BoNT(F2C), -(tag)₁-(X)₂-BoNT(V3C), -(tag)₁-(X)₂-BoNT(N4C), -(tag)₁-(X)₂-BoNT(K5C),
(d) -(tag)₁-(X)₃-BoNT(P1C), -(tag)₁-(X)₃-BoNT(F2C), -(tag)₁-(X)₃-BoNT(V3C), -(tag)₁-(X)₃-BoNT(N4C), -(tag)₁-(X)₃-BoNT(K5C),
(e) -(tag)₁-(X)₄-BoNT(P1C), -(tag)₁-(X)₄-BoNT(F2C), -(tag)₁-(X)₄-BoNT(V3C), -(tag)₁-(X)₄-BoNT(N4C), -(tag)₁-(X)₄-BoNT(K5C),
où, pour chacun des 25 modes de réalisation préférés énumérés ci-dessus, -(tag)₁ peut être aussi -(tag)₀, et/ou respectivement tous les résidus de cystéine introduits sur l'extrémité N-terminale sont aussi PEGylés.

8. Toxine botulique selon l'une des revendications précédentes, **caractérisée en ce que** la toxine botulique est une toxine botulique de type A, B ou C1.

9. Toxine botulique selon l'une des revendications précédentes, **caractérisée en ce que** la toxine botulique modifiée comprend au moins 20 %, de préférence 30 à 40 %, 50 à 70 % ou 75 à 95 % de l'activité biologique de la toxine botulique naturelle correspondante (non modifiée, native) et une stabilité accrue par rapport à cette dernière.

10. Toxine botulique selon l'une des revendications précédentes, **caractérisée en ce que** la chaîne légère modifiée est transloquée *in vivo* dans le cytosol des neurones moteurs.

11. Toxine botulique selon l'une des revendications précédentes, **caractérisée en ce que** la chaîne légère modifiée présente dans le cytosol des neurones moteurs une stabilité supérieure à celle de la toxine botulique native correspondante.

12. Composition pharmaceutique, la composition comprenant la toxine botulique modifiée selon l'une des revendications précédentes.

13. Composition pharmaceutique selon la revendication 12, la composition étant stabilisée sans addition d'albumine sérique humaine (HSA).

14. Composition pharmaceutique selon la revendication 12 ou 13, la composition se présentant sous une forme lyophilisée ou liquide et les deux formes étant appropriées, le cas échéant après dilution dans un solvant approprié, pour une injection IM.

15. Composition pharmaceutique selon la revendication 12, la composition étant destiné pour la thérapie des dystonies ainsi qu'à la suppression des rides du visage.


### SEQUENCE LISTING

<110> BioteCon Therapeutics GmbH
<120> PEGyliertes mutiertes Clostridium botulinum Toxin
<130> BIO-009 PCT
<140> unknown
   <141> 2007-03-15
<150> EP 06 005 300.6
   <151> 2006-03-15
<160> 16
<170> PatentIn version 3.3
<210> 1
   <211> 35
   <212> DNA
   <213> Artificial sequence
<220>
   <223> Primer
<400> 1
   accactccat ggcatttgtt aataaacaat ttaat 35
<210> 2
   <211> 67
   <212> DNA
   <213> Artificial sequence
<220>
   <223> Primer
<400> 2
   accaccagat ctatttaagg ccttggatcc acgcggaact aatgatttag ttttagaagt 60
   tattatc 67
<210> 3
   <211> 37
   <212> DNA
   <213> Artificial sequence
<220>
   <223> Primer
<400> 3
   accaccaggc cttaaatgat ttatgtatca aagttaa 37
<210> 4
   <211> 39
   <212> DNA
   <213> Artificial sequence
<220>
   <223> Primer
<400> 4
   accaccagat ctttacagtg gcctttctcc ccatccatc 39
<210> 5
   <211> 39
   <212> DNA
   <213> Artificial sequence
<220>
   <223> Primer
<400> 5
   accactccat ggcagttaca ataaataatt ttaattata 39
<210> 6
   <211> 85
   <212> DNA
   <213> Artificial sequence
<220>
   <223> Primer
<400> 6
   accactaggc cttggatcca cgtggaacca atgatttagt tttagaagtt attatcccac 60
   ttttacacat ttgtatctta tatac 85
<210> 7
   <211> 34
   <212> DNA
   <213> Artificial sequence
<220>
   <223> Primer
<400> 7
   accactaggc ctcaggaata tgtattgatg ttga 34
<210> 8
   <211> 33
   <212> DNA
   <213> Artificial sequence
<220>
   <223> Primer
<400> 8
   accaccagat ctttattcag tccacccttc atc 33
<210> 9
   <211> 40
   <212> DNA
   <213> Artificial sequence
<220>
   <223> Primer
<400> 9
   accactccat ggcaataaca attaacaact ttaattattc 40
<210> 10
   <211> 76
   <212> DNA
   <213> Artificial sequence
<220>
   <223> Primer
<400> 10
   gagaataggc cttggatcca cgcggaacta atgatttagt tttagaagtt attatccctc 60
   tatgacaaaa ttttgt 76
<210> 11
   <211> 33
   <212> DNA
   <213> Artificial sequence
<220>
   <223> Primer
<400> 11
   accactaggc cttagattgt agagagcttt tag 33
<210> 12
   <211> 44
   <212> DNA
   <213> Artificial sequence
<220>
   <223> Primer
<400> 12
   accaccagat ctttaagaaa taaatttcca aaaagatgaa gttg 44
<210> 13
   <211> 42
   <212> DNA
   <213> Artificial sequence
<220>
   <223> Primer
<400> 13
   gaggagaaat taactatggg atgtggatcg catcaccatc ac 42
<210> 14
   <211> 42
   <212> DNA
   <213> Artificial sequence
<220>
   <223> Primer
<400> 14
   gtgatggtga tgcgatccac atcccatagt taatttctcc tc 42
<210> 15
   <211> 3939
   <212> DNA
   <213> Artificial sequence
<220>
   <223> mutiertes Botulinum-Neurotoxin Typ A
<400> 15
   atgggatgtg gatcgcatca ccatcaccac catcatcacc atcactccat ggcatttgtt     60
   aataaacaat ttaattataa agatcctgta aatggtgttg atattgctta tataaaaatt    120
   ccaaatgcag gacaaatgca accagtaaaa gcttttaaaa ttcataataa aatatgggtt    180
   attccagaaa gagatacatt tacaaatcct gaagaaggag atttaaatcc accaccagaa    240
   gcaaaacaag ttccagtttc atattatgat tcaacatatt taagtacaga taatgaaaaa 300
   gataattatt taaagggagt tacaaaatta tttgagagaa tttattcaac tgatcttgga 360
   agaatgttgt taacatcaat agtaagggga ataccatttt ggggtggaag tacaatagat 420
   acagaattaa aagttattga tactaattgt attaatgtga tacaaccaga tggtagttat 480
   agatcagaag aacttaatct agtaataata ggaccctcag ctgatattat acagtttgaa 540
   tgtaaaagct ttggacatga agttttgaat cttacgcgaa atggttatgg ctctactcaa 600
   tacattagat ttagcccaga ttttacattt ggttttgagg agtcacttga agttgataca 660
   aatcctcttt taggtgcagg caaatttgct acagatccag cagtaacatt agcacatgaa 720
   cttatacatg ctggacatag attatatgga atagcaatta atccaaatag ggtttttaaa 780
   gtaaatacta atgcctatta tgaaatgagt gggttagaag taagctttga ggaacttaga 840
   acatttgggg gacatgatgc aaagtttata gatagtttac aggaaaacga atttcgtcta 900
   tattattata ataagtttaa agatatagca agtacactta ataaagctaa atcaatagta 960
   ggtactactg cttcattaca gtatatgaaa aatgttttta aagagaaata tctcctatct 1020
   gaagatacat ctggaaaatt ttcggtagat aaattaaaat ttgataagtt atacaaaatg 1080
   ttaacagaga tttacacaga ggataatttt gttaagtttt ttaaagtact taacagaaaa 1140
   acatatttga attttgataa agccgtattt aagataaata tagtacctaa ggtaaattac 1200
   acaatatatg atggatttaa tttaagaaat acaaatttag cagcaaactt taatggtcaa 1260
   aatacagaaa ttaataatat gaattttact aaactaaaaa attttactgg attgtttgaa 1320
   ttttataagt tgctatgtgt aagagggata ataacttcta aaactaaatc attagttccg 1380
   cgtggatcca aggccttaaa tgatttatgt atcaaagtta ataattggga cttgtttttt 1440
   agtccttcag aagataattt tactaatgat ctaaataaag gagaagaaat tacatctgat 1500
   actaatatag aagcagcaga agaaaatatt agtttagatt taatacaaca atattattta 1560
   acctttaatt ttgataatga acctgaaaat atttcaatag aaaatctttc aagtgacatt 1620
   ataggccaat tagaacttat gcctaatata gaaagatttc ctaatggaaa aaagtatgag 1680
   ttagataaat atactatgtt ccattatctt cgtgctcaag aatttgaaca tggtaaatct 1740
   aggattgctt taacaaattc tgttaacgaa gcattattaa atcctagtcg tgtttataca 1800
   tttttttctt cagactatgt aaagaaagtt aataaagcta cggaggcagc tatgttttta 1860
   ggctgggtag aacaattagt atatgatttt accgatgaaa ctagcgaagt aagtactacg 1920
   gataaaattg cggatataac tataattatt ccatatatag gacctgcttt aaatataggt 1980
   aatatgttat ataaagatga ttttgtaggt gctttaatat tttcaggagc tgttattctg 2040
   ttagaattta taccagagat tgcaatacct gtattaggta cttttgcact tgtatcatat 2100
   attgcgaata aggttctaac cgttcaaaca atagataatg ctttaagtaa aagaaatgaa 2160
   aaatgggatg aggtctataa atatatagta acaaattggt tagcaaaggt taatacacag 2220
   attgatctaa taagaaaaaa aatgaaagaa gctttagaaa atcaagcaga agcaacaaag 2280
   gctataataa actatcagta taatcaatat actgaggaag agaaaaataa tattaatttt 2340
   aatattgatg atttaagttc gaaacttaat gagtctataa ataaagctat gattaatata 2400
   aataaatttt tgaatcaatg ctctgtttca tatttaatga attctatgat cccttatggt 2460
   gttaaacggt tagaagattt tgatgctagt cttaaagatg cattattaaa gtatatatat 2520
   gataatagag gaactttaat tggtcaagta gatagattaa aagataaagt taataataca 2580
   cttagtacag atataccttt tcagctttcc aaatacgtag ataatcaaag attattatct 2640
   acatttactg aatatattaa gaatattatt aatacttcta tattgaattt aagatatgaa 2700
   agtaatcatt taatagactt atctaggtat gcatcaaaaa taaatattgg tagtaaagta 2760
   aattttgatc caatagataa aaatcaaatt caattattta atttagaaag tagtaaaatt 2820
   gaggtaattt taaaaaatgc tattgtatat aatagtatgt atgaaaattt tagtactagc 2880
   ttttggataa gaattcctaa gtattttaac agtataagtc taaataatga atatacaata 2940
   ataaattgta tggaaaataa ttcaggatgg aaagtatcac ttaattatgg tgaaataatc 3000
   tggactttac aggatactca ggaaataaaa caaagagtag tttttaaata cagtcaaatg 3060
   attaatatat cagattatat aaacagatgg atttttgtaa ctatcactaa taatagatta 3120
   aataactcta aaatttatat aaatggaaga ttaatagatc aaaaaccaat ttcaaattta 3180
   ggtaatattc atgctagtaa taatataatg tttaaattag atggttgtag agatacacat 3240
   agatatattt ggataaaata ttttaatctt tttgataagg aattaaatga aaaagaaatc 3300
   aaagatttat atgataatca atcaaattca ggtattttaa aagacttttg gggtgattat 3360
   ttacaatatg ataaaccata ctatatgtta aatttatatg atccaaataa atatgtcgat 3420
   gtaaataatg taggtattag aggttatatg tatcttaaag ggcctagagg tagcgtaatg 3480
   actacaaaca tttatttaaa ttcaagtttg tataggggga caaaatttat tataaaaaaa 3540
   tatgcttctg gaaataaaga taatattgtt agaaataatg atcgtgtata tattaatgta 3600
   gtagttaaaa ataaagaata taggttagct actaatgcat cacaggcagg cgtagaaaaa 3660
   atactaagtg cattagaaat acctgatgta ggaaatctaa gtcaagtagt agtaatgaag 3720
   tcaaaaaatg atcaaggaat aacaaataaa tgcaaaatga atttacaaga taataatggg 3780
   aatgatatag gctttatagg atttcatcag tttaataata tagctaaact agtagcaagt 3840
   aattggtata atagacaaat agaaagatct agtaggactt tgggttgctc atgggaattt 3900
   attcctgtag atgatggatg gggagaaagg ccactgtaa 3939
<210> 16
   <211> 1312
   <212> PRT
   <213> Artificial sequence
<220>
   <223> mutiertes Botulinum-Neurotoxin Typ A
<400> 16
   Met Gly Cys Gly Ser His His His His His His His His His His Ser
   1               5                   10                  15
   Met Ala Phe Val Asn Lys Gln Phe Asn Tyr Lys Asp Pro Val Asn Gly
               20                  25                  30
   Val Asp Ile Ala Tyr Ile Lys Ile Pro Asn Val Gly Gln Met Gln Pro
           35                  40                  45
   Val Lys Ala Phe Lys Ile His Asn Lys Ile Trp Val Ile Pro Glu Arg
       50                  55                  60
   Asp Thr Phe Thr Asn Pro Glu Glu Gly Asp Leu Asn Pro Pro Pro Glu
   65                  70                  75                  80
   Ala Lys Gln Val Pro Val Ser Tyr Tyr Asp Ser Thr Tyr Leu Ser Thr
                   85                  90                  95
   Asp Asn Glu Lys Asp Asn Tyr Leu Lys Gly Val Thr Lys Leu Phe Glu
               100                 105                 110
   Arg Ile Tyr Ser Thr Asp Leu Gly Arg Met Leu Leu Thr Ser Ile Val
           115                 120                 125
   Arg Gly Ile Pro Phe Trp Gly Gly Ser Thr Ile Asp Thr Glu Leu Lys
       130                 135                 140
   Val Ile Asp Thr Asn Cys Ile Asn Val Ile Gln Pro Asp Gly Ser Tyr
   145                 150                 155                 160
   Arg Ser Glu Glu Leu Asn Leu Val Ile Ile Gly Pro Ser Ala Asp Ile
                   165                 170                 175
   Ile Gln Phe Glu Cys Lys Ser Phe Gly His Glu Val Leu Asn Leu Thr
               180                 185                 190
   Arg Asn Gly Tyr Gly Ser Thr Gln Tyr Ile Arg Phe Ser Pro Asp Phe
           195                 200                 205
   Thr Phe Gly Phe Glu Glu Ser Leu Glu Val Asp Thr Asn Pro Leu Leu
       210                 215                 220
   Gly Ala Gly Lys Phe Ala Thr Asp Pro Ala Val Thr Leu Ala His Glu
   225                 230                 235                 240
   Leu Ile His Ala Gly His Arg Leu Tyr Gly Ile Ala Ile Asn Pro Asn
                   245                 250                 255
   Arg Val Phe Lys Val Asn Thr Asn Ala Tyr Tyr Glu Met Ser Gly Leu
               260                 265                 270
   Glu Val Ser Phe Glu Glu Leu Arg Thr Phe Gly Gly His Asp Ala Lys
           275                 280                 285
   Phe Ile Asp Ser Leu Gln Glu Asn Glu Phe Arg Leu Tyr Tyr Tyr Asn
       290                 295                 300
   Lys Phe Lys Asp Ile Ala Ser Thr Leu Asn Lys Ala Lys Ser Ile Val
   305                 310                 315                 320
   Gly Thr Thr Ala Ser Leu Gln Tyr Met Lys Asn Val Phe Lys Glu Lys
                   325                 330                 335
   Tyr Leu Leu Ser Glu Asp Thr Ser Gly Lys Phe Ser Val Asp Lys Leu
               340                 345                 350
   Lys Phe Asp Lys Leu Tyr Lys Met Leu Thr Glu Ile Tyr Thr Glu Asp
           355                 360                 365
   Asn Phe Val Lys Phe Phe Lys Val Leu Asn Arg Lys Thr Tyr Leu Asn
       370                 375                 380
   Phe Asp Lys Ala Val Phe Lys Ile Asn Ile Val Pro Lys Val Asn Tyr
   385                 390                 395                 400
   Thr Ile Tyr Asp Gly Phe Asn Leu Arg Asn Thr Asn Leu Ala Ala Asn
                   405                 410                 415
   Phe Asn Gly Gln Asn Thr Glu Ile Asn Asn Met Asn Phe Thr Lys Leu
               420                 425                 430
   Lys Asn Phe Thr Gly Leu Phe Glu Phe Tyr Lys Leu Leu Cys Val Arg
           435                 440                 445
   Gly Ile Ile Thr Ser Lys Thr Lys Ser Leu Val Pro Arg Gly Ser Lys
       450                 455                 460
   Ala Leu Asn Asp Leu Cys Ile Lys Val Asn Asn Trp Asp Leu Phe Phe
   465                 470                 475                 480
   Ser Pro Ser Glu Asp Asn Phe Thr Asn Asp Leu Asn Lys Gly Glu Glu
                   485                 490                 495
   Ile Thr Ser Asp Thr Asn Ile Glu Ala Ala Glu Glu Asn Ile Ser Leu
               500                 505                 510
   Asp Leu Ile Gln Gln Tyr Tyr Leu Thr Phe Asn Phe Asp Asn Glu Pro
           515                 520                 525
   Glu Asn Ile Ser Ile Glu Asn Leu Ser Ser Asp Ile Ile Gly Gln Leu
       530                 535                 540
   Glu Leu Met Pro Asn Ile Glu Arg Phe Pro Asn Gly Lys Lys Tyr Glu
   545                 550                 555                 560
   Leu Asp Lys Tyr Thr Met Phe His Tyr Leu Arg Ala Gln Glu Phe Glu
                   565                 570                 575
   His Gly Lys Ser Arg Ile Ala Leu Thr Asn Ser Val Asn Glu Ala Leu
               580                 585                 590
   Leu Asn Pro Ser Arg Val Tyr Thr Phe Phe Ser Ser Asp Tyr Val Lys
           595                 600                 605
   Lys Val Asn Lys Ala Thr Glu Ala Ala Met Phe Leu Gly Trp Val Glu
   610 615 620
   Gln Leu Val Tyr Asp Phe Thr Asp Glu Thr Ser Glu Val Ser Thr Thr
   625 630 635 640
   Asp Lys Ile Ala Asp Ile Thr Ile Ile Ile Pro Tyr Ile Gly Pro Ala
   645 650 655
   Leu Asn Ile Gly Asn Met Leu Tyr Lys Asp Asp Phe Val Gly Ala Leu
   660 665 670
   Ile Phe Ser Gly Ala Val Ile Leu Leu Glu Phe Ile Pro Glu Ile Ala
   675 680 685
   Ile Pro Val Leu Gly Thr Phe Ala Leu Val Ser Tyr Ile Ala Asn Lys
   690 695 700
   Val Leu Thr Val Gln Thr Ile Asp Asn Ala Leu Ser Lys Arg Asn Glu
   705 710 715 720
   Lys Trp Asp Glu Val Tyr Lys Tyr Ile Val Thr Asn Trp Leu Ala Lys
   725 730 735
   Val Asn Thr Gln Ile Asp Leu Ile Arg Lys Lys Met Lys Glu Ala Leu
   740 745 750
   Glu Asn Gln Ala Glu Ala Thr Lys Ala Ile Ile Asn Tyr Gln Tyr Asn
   755 760 765
   Gln Tyr Thr Glu Glu Glu Lys Asn Asn Ile Asn Phe Asn Ile Asp Asp
   770 775 780
   Leu Ser Ser Lys Leu Asn Glu Ser Ile Asn Lys Ala Met Ile Asn Ile
   785 790 795 800
   Asn Lys Phe Leu Asn Gln Cys Ser Val Ser Tyr Leu Met Asn Ser Met
   805 810 815
   Ile Pro Tyr Gly Val Lys Arg Leu Glu Asp Phe Asp Ala Ser Leu Lys
   820 825 830
   Asp Ala Leu Leu Lys Tyr Ile Tyr Asp Asn Arg Gly Thr Leu Ile Gly
   835 840 845
   Gln Val Asp Arg Leu Lys Asp Lys Val Asn Asn Thr Leu Ser Thr Asp
   850 855 860
   Ile Pro Phe Gln Leu Ser Lys Tyr Val Asp Asn Gln Arg Leu Leu Ser
   865 870 875 880
   Thr Phe Thr Glu Tyr Ile Lys Asn Ile Ile Asn Thr Ser Ile Leu Asn
   885 890 895
   Leu Arg Tyr Glu Ser Asn His Leu Ile Asp Leu Ser Arg Tyr Ala Ser
   900 905 910
   Lys Ile Asn Ile Gly Ser Lys Val Asn Phe Asp Pro Ile Asp Lys Asn
   915 920 925
   Gln Ile Gln Leu Phe Asn Leu Glu Ser Ser Lys Ile Glu Val Ile Leu
   930 935 940
   Lys Asn Ala Ile Val Tyr Asn Ser Met Tyr Glu Asn Phe Ser Thr Ser
   945 950 955 960
   Phe Trp Ile Arg Ile Pro Lys Tyr Phe Asn Ser Ile Ser Leu Asn Asn
   965 970 975
   Glu Tyr Thr Ile Ile Asn Cys Met Glu Asn Asn Ser Gly Trp Lys Val
   980 985 990
   Ser Leu Asn Tyr Gly Glu Ile Ile Trp Thr Leu Gln Asp Thr Gln Glu
   995 1000 1005
   Ile Lys Gln Arg Val Val Phe Lys Tyr Ser Gln Met Ile Asn Ile
   1010 1015 1020
   Ser Asp Tyr Ile Asn Arg Trp Ile Phe Val Thr Ile Thr Asn Asn
   1025 1030 1035
   Arg Leu Asn Asn Ser Lys Ile Tyr Ile Asn Gly Arg Leu Ile Asp
   1040 1045 1050
   Gln Lys Pro Ile Ser Asn Leu Gly Asn Ile His Ala Ser Asn Asn
   1055 1060 1065
   Ile Met Phe Lys Leu Asp Gly Cys Arg Asp Thr His Arg Tyr Ile
   1070 1075 1080
   Trp Ile Lys Tyr Phe Asn Leu Phe Asp Lys Glu Leu Asn Glu Lys
   1085 1090 1095
   Glu Ile Lys Asp Leu Tyr Asp Asn Gln Ser Asn Ser Gly Ile Leu
   1100 1105 1110
   Lys Asp Phe Trp Gly Asp Tyr Leu Gln Tyr Asp Lys Pro Tyr Tyr
   1115 1120 1125
   Met Leu Asn Leu Tyr Asp Pro Asn Lys Tyr Val Asp Val Asn Asn
   1130 1135 1140
   Val Gly Ile Arg Gly Tyr Met Tyr Leu Lys Gly Pro Arg Gly Ser
   1145 1150 1155
   Val Met Thr Thr Asn Ile Tyr Leu Asn Ser Ser Leu Tyr Arg Gly
   1160 1165 1170
   Thr Lys Phe Ile Ile Lys Lys Tyr Ala Ser Gly Asn Lys Asp Asn
   1175 1180 1185
   Ile Val Arg Asn Asn Asp Arg Val Tyr Ile Asn Val Val Val Lys
   1190 1195 1200
   Asn Lys Glu Tyr Arg Leu Ala Thr Asn Ala Ser Gln Ala Gly Val
   1205 1210 1215
   Glu Lys Ile Leu Ser Ala Leu Glu Ile Pro Asp Val Gly Asn Leu
   1220 1225 1230
   Ser Gln Val Val Val Met Lys Ser Lys Asn Asp Gln Gly Ile Thr
   1235 1240 1245
   Asn Lys Cys Lys Met Asn Leu Gln Asp Asn Asn Gly Asn Asp Ile
   1250 1255 1260
   Gly Phe Ile Gly Phe His Gln Phe Asn Asn Ile Ala Lys Leu Val
   1265 1270 1275
   Ala Ser Asn Trp Tyr Asn Arg Gln Ile Glu Arg Ser Ser Arg Thr
   1280 1285 1290
   Leu Gly Cys Ser Trp Glu Phe Ile Pro Val Asp Asp Gly Trp Gly
   1295 1300 1305
   Glu Arg Pro Leu
   1310